# EUROPEAN PATENT APPLICATION

(11) **EP 1 130 095 A1**
(43) Date of publication of application: **05.09.2001**
(21) Application number: 00400427.1
(22) Date of filing: 15.02.2000
(51) Int. Cl.: C12N 15/12, C07K 14/705, C07K 16/28, C12Q 1/68, G01N 33/68

(54) **Control of membrane traffic**

(71) Applicant: Institut Curie, 75248 Paris Cedex 05 (FR); CENTRE NATIONAL DE LA RECHERCHE SCIENTIFIQUE, 75016 Paris Cédex (FR)
(72) Inventor: Galli, Thierry, 75005 Paris (FR); Louvard, Daniel, 92330 Suresnes (FR); Martinez, Sonia, 75005 Paris (FR)
(74) Representative: Peaucelle, Chantal

(57) **Abstract**

The present invention relates to the control of membrane traffic inside cells, such as those involving fusion events and in particular those involving exocytic events. It more particularly relates to N-terminal fragments of TeNT-insensitive VAMP, and to TeNT-insensitive VAMP deleted from such fragments, and to the biological applications of such products, notably for controlling TeNT-resistant pathways such as neurite outgrowth and cell motility.

## Description

The present invention relates to the control of membrane traffic inside cells, such as those involving fusion events and in particular those involving exocytic events. It more particularly relates to the positive and negative regulation of any tetanus neurotoxin (TeNT) -resistant pathway inside a cell, such as any pathway involving the activity of a TeNT-insensitive VAMP (vesicular-associated membrane protein). The present invention indeed provides with new products and new means for controlling such a membrane traffic inside a cell, and notably for controlling any cell activity involving a TeNT-insensitive a VAMP such as TI-VAMP (tetanus neurotoxin-insensitive vesicle-associated membrane protein).

Many different VAMP have been described in the prior art. These VAMP are receptors expressed by cell vesicles, and are known to be involved in exocytic events. But very little was known on how to control the activity of TeNT-insensitive VAMP, and of TI-VAMP in particular.

The present invention provides with new products and means solving this problem, and further gives for the first time the demonstration that such new products and means are efficient in controlling traffic membrane in non-epithelial cells, and notably in neuronal cells and in tumoral cells.

The present invention thus encompasses any isolated polypeptide of which sequence corresponds to a sequence chosen among the group consisting of the N-terminal domain sequences of any TeNT-insensitive VAMP such as TI-VAMP, and the conservative fragments and variants thereof. Such isolated polypeptide do not comprise any coiled coil motif.

It more particularly relates to any isolated polypeptide of which sequence corresponds to a sequence chosen among the group consisting of a sequence corresponding to SEQ ID N°2, a sequence corresponding to SEQ ID N°20, the sequences corresponding to any conservative fragment of SEQ ID N°2, the sequences corresponding to any conservative fragment of SEQ ID N°20, the sequences corresponding to any conservative variant of SEQ ID N°2, the sequences corresponding to any conservative variant of SEQ ID N°20.

These polypeptides will be referred to herein as the Nter polypeptides.

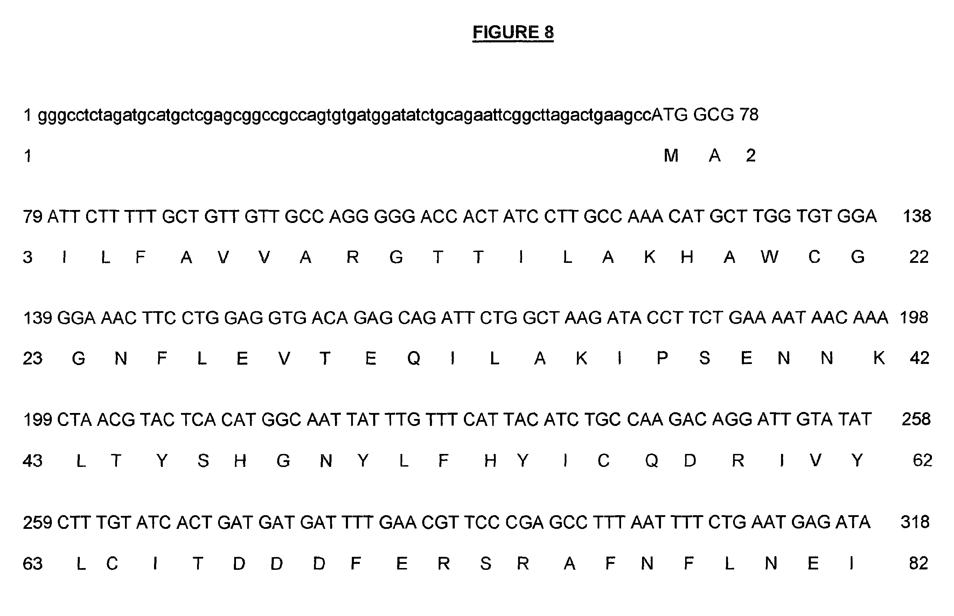

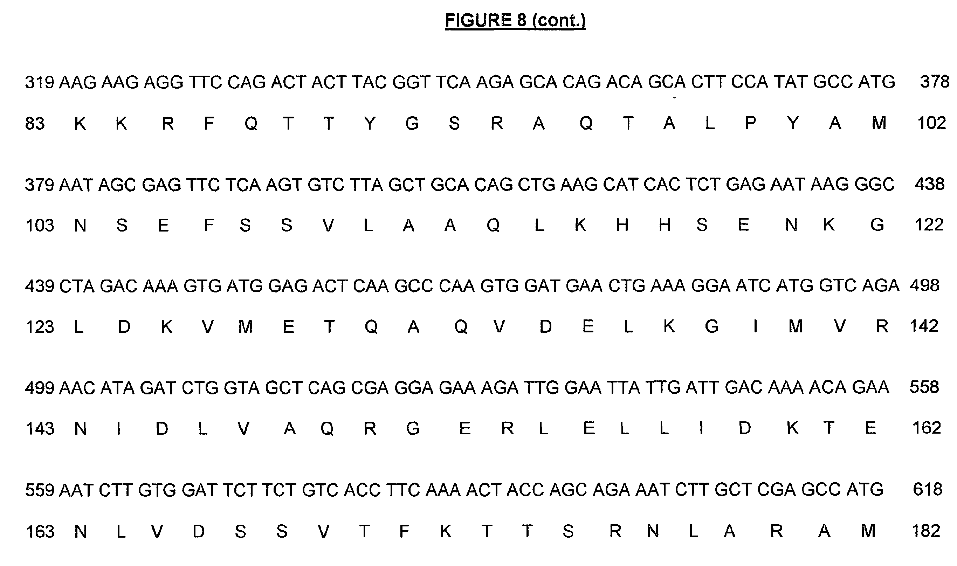

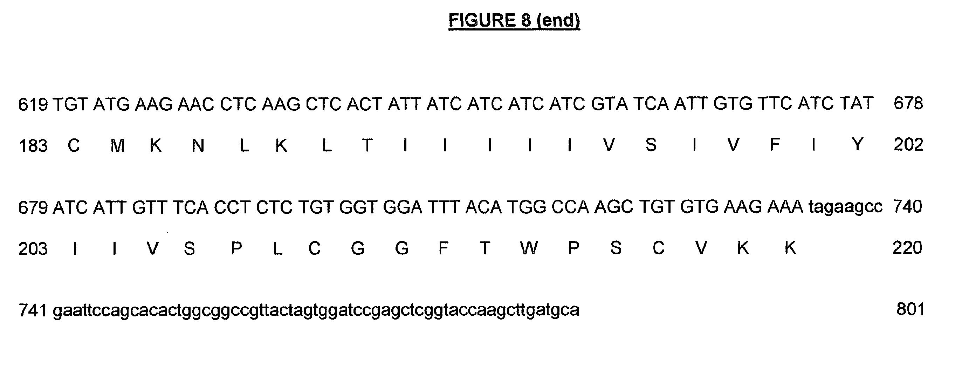

SEQ ID N°2 refers to an isolated polypeptide of which sequence corresponds to the first 120 aminoacids (M¹-N¹²⁰ en Figure 8) of human normal TI-VAMP (SEQ ID N°6). SEQ ID N°2 is also referred to as Nter in the below examples. SEQ ID N°20 refers to an isolated polypeptide of which sequence corresponds to the first 101 aminoacids (M¹-A¹⁰¹ on Figure 8) of human normal TI-VAMP (SEQ ID N°6).

As these aa sequences, their corresponding polynucleotidic sequences, and the conservative fragment sequences thereof are from normal human origin, they and their human variants are considered as corresponding to one of the best modes of the invention. Whereas the complete sequence of TI-VAMP has been previously described, N-terminal domain (*i.e.* N-terminal region which excludes any coiled coil motif) had no known function up to the present invention. As there was no aim at it, the skilled person would then not have seriously encompased to produce a polypeptide limited to such a N-terminal domain, or to produce a polypeptide limited to the coiled coils plus C-terminal region of TI-VAMP. The present invention describes for the first time a biological function for these polypeptides : they are capable of regulating a membrane traffic, and in particular TeNT-resistant pathways. According to a further remarquable aspect, they are capable of exerting these capacities on neuronal maturation and/or differentiation (neurite outgrowth) and on the motility of tumoral cells.

By « conservative » product (polypeptide or polynucleotidic fragment, variant polypeptide or polynucleotide), it is meant in the present application that this product is capable of showing under physiological conditions at least one of the biological properties shown by a « parent» product (SEQ ID N°2 -also referred to as Nter in the below examples -, and SEQ ID N°20 -from M¹ to A¹⁰¹ on figure 8).

Such biological properties notably include the capacity of inhibiting a membrane traffic pathway inside a cell, the capacity of inhibiting a function involving at least one TeNT-insensitive pathway, the capacity of inhibiting the activity of a TeNT-insensitive VAMP such as TI-VAMP, the capacity of inhibiting a function involving a fusion function or an exocytose function of a cell, a capacity of inhibiting neurite outgrowth, a capacity of inhibiting the motility of cells such as metastasis-forming cells (tumoral cells). Any cell enabling the skilled person to perform the desired assay is appropriate. Preferred cells are cells of animal or human origin, including cell lines. Cells of special interest for industrial applications namely include neuronal cells and tumoral cells, as from primary cultures, as well as from cell lines such a PC12 cells for neuronal cells, or MDCK, HeLAa, CACO-2, NIH-3-T3 cells for tumoral cells.

By physiological conditions, it is herein meant in *vivo* conditions, or *in vitro* conditions mimicking the *in vivo* ones ; typically, appropriate physiological conditions comprise conditions of medium composition, atmosphere, pH which are adequate to the cells that may be involved in the assay, and notably to animal or human cells.

By a « variant » product, it is herein meant any product which corresponds to the « parent» product after one or several of its elementary components (aminoacid, or when the case arises, nucleotide) has (have) been deleted and/or inverted and/or substituted, in so far this variant product has retained at least one of the biological properties shown by the « parent» product as above-defined. When reference is made to any aminoacid or polypeptide deletion/inversion/substitution, it has to be considered in the context of the universal genetic code and its redundancies. A variant product thus notably encompasses any product of which sequence shows with the parent product (SEQ ID N°2 or N°20 in the case of polypeptides; SEQ ID N°1 or SEQ ID N°19 for their respective DNA sequences), over the entire length of this parent product sequence, an homology of at least about 50%, particularly of at least about 60%, more particularly of at least about 70%, preferably of at least about 80%, more preferably of at least about 90%, the most preferred being of at least about 95%. A variant product also encompasses any conservative fragments of such products. While the invention is more particularly illustrated for human cells, variant products which can be found in other cell types or in other species, and notably among animals, are thus encompassed by the present invention.

By "TeNT-resistant" or "TeNT-insensitive pathway or receptor, the present application means that said pathway or said receptor is still active when the cell wherein said pathway takes places or said receptor is expressed is placed into contact with tetanus neurotoxin under exposure to the TeNT produced by the clostridium tetanii in the case of sensitive cells such as neurons, or of transfection of the cDNA coding for the light chain of TeNT in all cases.

In the present application, the expressions "inhibiting", "stimulating" are meant as statistically significant negative or positive difference, and thus encompasses "blocking" and, respectively, "inducing".

According to a complementary and corresponding aspect, the invention also relates to any isolated polypeptide of which sequence corresponds to a sequence chosen among the group consisting of the TeNT-insensitive VAMP sequences, such as TI-VAMP sequence, deleted from their respective N-terminal domains, provided that the resulting "deleted" polypeptide is still capable of showing an activity of the coiled coil type (*i.e.* the SNARE motif activity is not disrupted), namely is still capable of binding to at least one of the target SNARE (target Soluble N-ethylmaleide-sensistive fusion protein Attachment Receptor) of the corresponding complete VAMP (t-SNARE of TI-VAMP namely comprise SNAP 25, SNAP23, syntaxin1, syntaxin3). Coiled coils of VAMP can be predicted by computer programs such as COILS. These polypeptides will be referred to herein as the « deleted » polypeptides (also refered to as □Nter-TI-VAMP in the below examples). The invention more particularly relates to polypeptides of which sequence corresponds to SEQ ID N°6 of which N-terminal domain has been deleted from at least one Nter polypeptide according to the invention. Examples of such "deleted polypeptides" include those of which sequence corresponds to a sequence chosen among the group consisting of SEQ ID N°4, the sequences corresponding to any conservative variant of SEQ ID N°4, and the sequences corresponding to any conservative fragment of SEQ ID N°4.

SEQ ID N°6 refers to the complete aminoacid sequence of human TI-VAMP (see figure 8).

SEQ ID N°4 refers to the aminoacid sequence of an isolated polypeptide of which sequence corresponds to the aminoacid sequence of human TI-VAMP deleted from the first 101 N-terminal aminoacids (*i.e.* it corresponds to M¹⁰² to the end of SEQ ID N°6).

As SEQ ID N°4, its corresponding polynucleotidic sequence (SEQ ID N°3), and the conservative fragments thereof are from normal human origin, they and their human variants are considered as one of the best modes of the invention.

The biological properties shown by a polypeptide of SEQ ID N°4 correspond to reversed properties of SEQ ID N°2 or 20. They notably include the capacity of stimulating a membrane traffic pathway inside a cell, the capacity of stimulating a function involving at least one TeNT-resistant pathway, the capacity of stimulating the activity of a TeNT-insensitive VAMP such as TI-VAMP, the capacity of stimulating a function involving a fusion function or an exocytose function of a cell, a capacity of stimulating neurite outgrowth, a capacity of stimulating the motility of cells such as metastasis-forming cells. « variant » is as above-defined.

Both the Nter and the "deleted" polypeptides of the invention are advantageously associated to any product enabling their passage inside a cell, such as an animal cell, a human cell, a plant cell, an eucaryotic cell, a protiste.
So as to make it penetrate into a cell, a polypeptide of the invention can be *e.g.* chemically modified, for examples by esterification by addition of a lipidic tail, and/or can be associated to a carrier-delivery system such as liposomes.

The invention further provides with products derived from the Nter polypeptides of the invention. It thus relates to any product chosen among the group consisting of the monoclonal antibodies capable of binding to a polypeptide according to the invention, and the Fab, F(ab')₂, CDS fragments thereof. These products will be referred to as the "binding" products of the invention. Production of monoclonal antibodies is of common knowledge to the skilled person (notably : Köhler and Milstein procedure). Such monoclonal antibodies advantageously do not bind to a « deleted » polypeptide of the invention. The invention more particularly encompasses those products which are capable of inhibiting under physiological conditions at least one of the biological properties a Nter polypeptide according to the invention can show. Such biological properties of a Nter polypeptide namely comprise the inhibition of the formation of complexes between a TeNT-insensitive VAMP such as TI-VAMP, and a target SNARE (*e.g.* SNAP25, SNAP23, syntaxin1, syntaxin3 for TI-VAMP).

According to further aspect of the invention, sharing corresponding features with the polypeptides of the invention, there are provided polynucleotides of which sequence codes for the Nter polypeptides according to the invention, polynucleotides of which sequence codes for the "deleted" polypeptides according to the invention, and polynucleotides of which sequence codes for any binding product according to the invention.

As Nter polynucleotides, the invention more particularly relates to any isolated polynucleotide of which sequence corresponds to a sequence chosen of which sequence corresponds to a sequence chosen among the group consisting of a sequence corresponding to SEQ ID N°1, a sequence corresponding to SEQ ID N°19, the sequence corresponding to any conservative fragment of SEQ ID N°1, the sequence corresponding to any conservative variant of SEQ ID N°1, the sequence corresponding to any conservative fragment of SEQ ID N°19, the sequence corresponding to any conservative variant of SEQ ID N°19.

SEQ ID N°1 refers to the DNA sequence coding for SEQ ID N°2 (first N-terminal 120 aa of TI-VAMP, *i.e.* from M¹ to N¹²⁰, that is to say from position 73 to position 432 on the TI-VAMP DNA Sequence, see figure 8).

SEQ ID N°19 refers to the DNA sequence coding for SEQ ID N°20 (first N-terminal 101 aa of TI-VAMP, *i.e.* from M¹ to position 101, that is to say from position 73 to position 375 on the TI-VAMP DNA sequence, see figure 8).

As "deleted" polynucleotides, the invention more particularly relates to any isolated polynucleotide of which sequence corresponds to a sequence chosen among the group consisting of the SEQ ID N°5 sequences of which 5' domain has been deleted from at least one Nter polynucleotide according to the invention. The "deleted" polynucleotides of the invention namely comprise any isolated polynucleotide of which sequence corresponds to a sequence chosen among the group consisting of a sequence corresponding to SEQ ID N°3, the sequences corresponding to any conservative fragment of SEQ ID N°3, the sequences corresponding to any conservative variant of SEQ ID N°3. SEQ ID N°5 refers to the complete DNA sequence of human TI-VAMP (see figure 8).

SEQ ID N°3 refers to the DNA sequence of an isolated polynucleotide of which sequence corresponds to a sequence coding for M¹⁰² to the end of SEQ ID N°6, that is to say corresponds to a position 376-732 DNA fragment of SEQ ID N°5.

All of these polynucleotides are particularly useful for transfection into a cell such as an animal cell, a human cell, a cell line, a plant cell, an eucaryotic cell, a protist. When appropriate, transfection vectors, such as plasmids or retrovirus, which comprise at least one polynucleotide according to the invention, can be constructed by the skilled person. Preferably, said at least one polynucleotide is comprised in said transfection vector in a coding position.

Appropriate promotors namely comprise those of Cytomegalovirus or of TI-VAMP himself (Synaptobrevin like gene 1, Matarazzo *et al.* Gene 1999, 240:233-238).

When the transfection vector comprises at least one Nter polynucleotide of the invention, it is herein referred to as "Nter" transfection vector.

When the transfection vector comprises at least one "deleted" polynucleotide of the invention, it is herein referred to as "deleted" transfection vector. Examples of such vectors and of such transfection are illustrated in the below examples.

The invention further encompasses any cell that has been genetically engineered cell so as to comprise at least one product chosen among :
- the group consisting of the Nter polynucleotides according to the invention, the Nter transfection vectors according to the invention, this group being referred to as the "Nter" cells of the invention, or
- the group consisting of the "deleted" polynucleotides according to the invention, the transfection vectors according to the invention, this group being referred to as the "deleted" cells of the invention.

Preferred Nter and "deleted" cells of the invention produce Nter, or respectively, "deleted" polypeptides according to the invention.

Preferred cells are protist cells, eucaryotic cells, human cells, animal cells such as animal ovary cells, human or animal neuronal cells, tumoral cells.

According to a further complementary aspect of the invention, there are provided new products enabling the isolation of the polynucleotides of the invention.

The invention indeed encompasses any pair of pair of oligonucleotides characterized in that it is capable under standard PCR conditions to amplify at least one Nter polynucleotide according to the invention.

Such oligonucleotides pairs namely comprise the SEQ ID N°11, SEQ ID N°12 pair (see example 1 below).

The invention correspondingly also encompasses any pair of oligonucleotides characterized in that it is capable under standard PCR conditions to amplify at least one "deleted" polynucleotide according to the invention.

Such oligonucleotide pairs namely comprise the SEQ ID N°15, SEQ ID N°16 pair (see example 1 below).

By standard PCR conditions, it is herein meant PCR conditions that enable the skilled person to amplify from a polynucleotidic population the desired polynucleotide at the desired purity or specificity. The adjustement of such conditions are of common knowledge. Examples of appropriate conditions include the calculation of the melting temperature of the primers according to common knowledge in the art, and a choice of MgCl₂ concentration so as to establish the desired stringency.

These oligonucleotidic pairs of the invention allow the isolation of Nter polynucleotides according to the invention, and respectively "deleted" polynucleotides according to the invention. This isolation can be performed from a polynucleotide population extracted from cells such as human cells. A standard way to proceed is to place at least one oligonucleotidic pair according to the invention into contact with such a polynucleotide population under conditions appropriate to PCR amplification as above-defined. This method of the invention namely allows the skilled person to obtain any desired variant and/or fragment of Nter polynucleotides and "deleted" polynucleotides of the invention. It further enables to isolate mutant non-functional forms of human or animal Nter and "deleted" polynucleotides of the invention.

The present invention thus encompasses any isolated polynucleotide which is such as obtained by using on a polynucleotide population at least one pair of oligonucleotides according to the invention. Appropriate polynucleotide populations namely comprise those extracted from human or animal cells, from human or animal normal cells, from human or animal pathological cells, from human or animal nerve or neuronal cells, from human or animal tumoral cells. An easy way to proceed is via PCR.

Once such polynucleotides are isolated, it is of common knowledge to the skilled person to deduce from them the aminoacid sequences that correspond to them.

According to aspect of particular interest for industrial application, the invention provides with pharmaceutical compositions which comprise at least one product chosen among the group consisting of the Nter polypeptides according to the invention, the Nter polynucleotides according to the invention, the Nter transfection vectors according to the invention, and the Nter cells according to the invention. Such a pharmaceutical composition may notably be a drug. In this case, the drug of the invention may comprise said at least one product in a quantity appropriate for inhibiting a cell membrane traffic, and/or a function involving a TeNT-insensitive pathway, and/or a function involving a TeNT-resistant VAMP such as TI-VAMP, and/or a cell fusion or cell exocytic function, and/or the formation of complexes involving a TeNT-insensitive VAMP such as TI-VAMP, and/or a cell undesired motility such as the motility of cells susceptible of forming metastasis. Pharmaceutical compositions of the invention thus notably comprise antitumoral drugs. It also relates to the use of such products for the production of such a pharmaceutical composition.

According to another aspect of particular interest for industrial application, the invention provides with pharmaceutical composition comprising at least one product chosen among the group consisting of the "deleted" polypeptides according to the invention, the "deleted" polynucleotides according to the invention, the "deleted" transfection vectors according to the invention, and the "deleted" cells according to the invention. Such a pharmaceutical composition may notably be a drug. In this case, the drug of the invention may comprise said at least one product in a quantity appropriate for stimulating a cell membrane traffic, and/or a function involving a TeNT-resistant pathway, and/or a function involving a TeNT-insensitive VAMP such as TI-VAMP, and/or a cell fusion or a cell exocytic function, and/or the formation of complexes involving a TeNT-insensitive VAMP such as TI-VAMP, and/or a neurite outgrowth (axonal and/or dendritic outgrowth), and/or a neuronal maturation, and/or a neuronal differentiation, and/or appropriate for stimulating memory and/or learning. Pharmaceutical compositions of the invention thus notably comprise drugs intended for the therapy and/or palliation and/or prevention of spinal cord trauma.

It also relates to the use of such products for the production of such a pharmaceutical composition.

The pharmaceutical compositions and drugs of the invention may further comprise any additive, co-agent, buffer appropriate to the application for which it is intended. The formulation of such compositions and drugs can be chosen and adjusted by the skilled person in function of the precise reactive agent chosen (polypeptide/polynucleotide/transfection vector/cell), in function of the additives co-agents and/or buffer that have been chosen, and has to take into account the administration route that is desired (topical, oral, injection, subcutaneous, implant, patch, spray, transfection, etc.).

The invention also provides with new means, making use of the new products of the invention, and sharing with them the same or corresponding feature of the inhibition function of which is capable the N-terminal domain of a TeNT-insensitive VAMP such as TI-VAMP.

The invention notably provides with a method for identifying a pharmaceutical agent capable of stimulating a cell function chosen among the group consisting of the cell functions involving a membrane traffic, the cell functions involving a TeNT-resistant pathway, the cell functions involving the formation of complexes with a TeNT-insensitive VAMP such as TI-VAMP, the cell functions involving at least one TeNT-insensitive VAMP such as TI-VAMP, the cell functions involving a fusion, or an exocytic event, the cell functions involved in neurite outgrowth, in neuronal maturation, in neuronal differentiation, in memory ability, in learning capacity, characterized in that it comprises at least one step chosen among the group consisting of:
- the identification of an agent that is capable under physiological conditions of blocking or diminishing the inhibition effect that is observed when the cytoplasm of said cell is placed into contact with at least one product chosen among the group consisting of the Nter polypeptides according to the invention, the Nter polynucleotides according to the invention, the Nter transfection vectors according to the invention, the Nter cells according to the invention, under conditions appropriate to cell membrane trafficking,
- the identification of an agent that is capable under physiological conditions to compete with a "binding" product according to the invention for binding to at least one Nter polypeptide of the invention,
- the identification of an agent that is capable under physiological conditions of stimulating the formation of complexes involving at least one "deleted" polypeptide according to the invention, and at least one t-SNARE (such as SNAP25, SNAP23, syntaxinl, syntaxin3 for TI-VAMP),
- the identification of an agent that is capable under physiological conditions of exerting an additional or synergic effect on the cell function that is observed when the cytoplasm of said cell is placed into contact with at least one product chosen among the group consisting of the "deleted" polypeptides according to the invention, the "binding" products according to the invention, the "deleted" polynucleotides according to the invention, the "deleted" transfection vectors according to the invention, the "deleted" cells according to the invention, under conditions appropriate to cell membrane trafficking.

The invention also provides with a method for identifying a pharmaceutical agent capable of inhibiting a cell function chosen among the group consisting of the cell functions involving a membrane traffic, the cell functions involving a TeNT-resistant pathway, the cell functions involving the formation of complexes with a TeNT-insensitive VAMP such as TI-VAMP, the cell functions involving at least one TeNT-insensitive VAMP such as TI-VAMP, the cell functions involving a fusion, or an exocytic event, the cell functions involved in cell motility, the cell functions involved in the formation of metastasis, characterized in that it comprises at least one step chosen among the group consisting of:
- the identification of an agent that is capable under physiological conditions of stimulating the inhibition effect that is observed when the cytoplasm of said cell is placed into contact with at least one product chosen among the group consisting of the Nter polypeptides according to the invention, the Nter polynucleotides according to the invention, the Nter transfection vectors according to the invention, the Nter cells according to the invention, under conditions appropriate to cell membrane trafficking,
- the identification of an agent that is capable under physiological conditions of inhibiting the formation of complexes involving at least one "deleted" polypeptide according to the invention, and at least one t-SNARE (such as SNAP25, SNAP23, syntaxi1, syntaxin3 for TI-VAMP),
- the identification of an agent that is capable under physiological conditions of exerting an inhibitory effect on the cell function that is observed when the cytoplasm of said cell is placed into contact with at least one product chosen among the group consisting of the "deleted" polypeptides according the invention, the "binding" products according to the invention, the "deleted" polynucleotides according to the invention, the "deleted" transfection vectors according to the invention, the "deleted" cells according to the invention, under conditions appropriate to cell membrane trafficking,
- the identification of an agent that is capable under physiological conditions to lyse a "binding" product of the invention which is capable of inhibiting at least one of the biological properties a Nter polypeptide can show, as above-defined.

For the identification methods of the invention, any appropriate cell is convenient. It notably includes human and animal, normal and pathological cells, neuronal cells, tumoral cells.

Said identification may be performed by any means the skilled person find appropriate. This namely includes the screening of chemical and/or biological libraries for an agent having the desired capacity. Screening in function of the capacity of the test agent to bind onto a t-SNARE (such as SNAP25, SNAP23, syntaxinl and/or syntaxin3 for TI-VAMP) may involve the association of the test products with a tag such as GST, GFP the immobilization of at least one appropriate t-SNARE on a solid support such as a membrane, and the detection by antibodies of those test products which are retained onto said solid support (see *e.g.* overlay assay in the above example 1). Said identification may also be performed by ELISA techniques.

The present invention also encompasses any pharmaceutical agent such as obtained by a method of identification according to the invention. These agents are regulatory agents : the ones obtained by the first method are stimulation agents, the ones obtained by the latter method are inhibition agents.

A further aspect of industrial interest relates to the use of at least one product chosen among the group consisting of the Nter polypeptides according to the invention, the Nter polynucleotides according to the invention, the Nter transfection vectors according to the invention, the Nter cells according to the invention, the inhibition pharmaceutical agents according to the invention, for the production of a drug intended for at least one effect chosen among the group consisting of inhibiting a cell membrane traffic, inhibiting a vesicular transport, inhibiting a function involving at least one TeNT-resistant pathway, inhibiting a function involving a TeNT-insensitive VAMP such as TI-VAMP, inhibiting the formation of complexes involving at least one TeNT-insensitive VAMP such as TI-VAMP and at least one t-SNARE (such as SNAP25, SNAP23, syntaxin 1, syntaxin 3, for TI-VAMP), inhibiting a cell motility such as the motility of cells susceptible of forming metastasis.

The present invention further relates to the use of at least one product chosen among the group consisting of the "deleted" polypeptides according to the invention, the "binding" products according to the invention, the "deleted" polynucleotides according to the invention, the "deleted" transfection vectors according to the invention, the "deleted" cells according to the invention, the stimulation pharmaceutical agents according to the invention, for the production of a drug intended for at least one effect chosen among the group consisting of stimulating a cell membrane traffic, stimulating a vesicular transport, stimulating a function involving at least one TeNT-resistant pathway, stimulating a function involving a TeNT-insensitive VAMP such as TI-VAMP, stimulating the formation of complexes involving at least one TeNT-insensitive VAMP such as TI-VAMP and at least one t-SNARE (such as, for the TI-VAMP : SNAP25, SNAP23, syntaxin 1, syntaxin 3), stimulating axonal and/or dendritic outgrowth, stimuling, neuronal maturation, stimulating neuronal differentiation, stimulating memory and/or learning capacity, curing and/or palliating and/or preventing spinal cord trauma, curing and/or palliating and/or preventing neuro-degenerative disorders, curing and/or palliating and/or preventing sclerosis.

Another aspect of the invention that is of industrial interest relates to a method for diagnosing an undesired state, and/or for assessing the efficiency of a medical treatment, and/or for assessing the evolution of an undesired state, characterized in that it comprises at least one step chosen among the group consisting of:
- the detection, in a biological sample, of a presence level significantly different from the standard level for at least one product chosen among the group consisting of the "deleted" polypeptides according to the invention, the "binding" products according to the invention, the "deleted" polynucleotides according to the invention, the "deleted" transfection vectors according to the invention, the "deleted" cells according to the invention (this step will be referred to as step a.),
- the detection in a biological sample of a level of expression for Nter polypeptide according to the invention significantly different from the standard expression level (step b.).

Said biological sample may notably be a peripheral blood, tissue or biological liquid sample. It remarquably may correspond to a neuronal cell sample, or to a tumoral cell sample.

Said undesired state may correspond to any state chosen among the group consisting of the states involving the disregulation of a cell membrane traffic, of a cell TeNT-resistant pathway, of a cell function involving the activity of a TeNT-insensitive VAMP such as TI-VAMP, of the formation of complexes between a TeNT-insensitive VAMP such as TI-VAMP and a t-SNARE (SNAP 25, SNAP 23, syntaxin1, syntaxin3 are t-SNARE for TI-VAMP), of a fusion or an exocytic event. It remarquably may correspond to a state involving the disregulation of the differentiation and/or of the maturation of neuronal cells (disregulation of neurite outgrowth) : this is for example the case of spiral cord trauma, and also of neuro-degenerative disorders, and of certain sclerosis. Said undesired state may also correspond to a state involving the disregulation of the motility of the cells, as this is in particularly the case for tumoral cells disseminating so as to form metastasis.

When the presence level detected according to the above-defined step a. is significantly superior to the standard level, and/or when the expression level detected in the above-defined step b. is significantly inferior to the standard expression level; the state concerned is disregulated in the sens of an excess of stimulation (superior level in step a.) or of a lack of inhibition (inferior level in step b.). This may be the case *e.g.* of proliferating and dissiminating tumoral cells.

When the presence level detected according to the above-defined step a. is significantly inferior to the standard level, and/or when the expression level detected in the above-defined step b. is significantly superior to the standard expression level, the state concerned is disregulated in the sens of a lack of stimulation (inferior level in step a.) or of an excess of inhibition (superior level in step b.). This may be the case *e.g.* of non- or insufficienty maturing and/or differentiating neuronal cells.

The invention also encompasses the kits for implementing this method of the invention. Such kits may comprise at least one of said product of the invention, possibly together with appropriate visualisation agent such as GFP or GST tag.

The present invention further relates to a method for identifying a compound capable of assuming the function of a biological effector of a TeNT-insensitive VAMP such as TI-VAMP, characterized in that it comprises at least one step chosen among the group consisting of:
- the detection of a compound which is capable under physiological conditions of binding to a Nter polypeptide according to the invention,
- the detection of a compound which is capable under physiological conditions of diminishing the inhibition effect that is observed when the cytoplasm of a cell expressing said TeNT-insensitive VAMP is placed into contact with at least one product chosen among the group consisting of the Nter polypeptides according to the invention, the Nter polynucleotides according the invention, the Nter transfection vectors according to the invention, the Nter cells according to the invention, under conditions appropriate to cell membrane trafficking,
- the detection of a compound which is capable under physiological conditions of exerting an additional or synergic effect on the cell function that is observed when the cytoplasm of a cell expressing a TeNT-insensitive VAMP such as TI-VAMP is placed into contact with at least one product chosen among the group consisting of the "deleted" polypeptides according to the invention, the "binding" products according to the invention, the "deleted" polynucleotides according to the invention, the "deleted" transfection vectors according to the invention, the "deleted" cells according to the invention, under conditions appropriate to cell membrane trafficking.

Any cell may be appropriate. It remarquably includes human or animal neuronal and tumoral cells. Said detection step may be performed according to the common knowledge of the persons skilled in the identification of a compound having the desired capacity. It namely includes screening of chemical and/or biological libraries.

An effector compound such as identified by the method of the invention is of particular use for moduling the activity of its TeNT-insensitive VAMP receptor. It may *e.g.* be used as a co-agent in the applications described herein.

Other and further characteristics, advantages, and variant embodiments of the invention can be found by the skilled persons from the below-given examples. These examples are given for illustration purposes only, they do not limit the scope of the invention. In particular, while these examples illustrate the invention for the human normal TeNT-insensitive VAMP TI-VAMP, the skilled person can proceed similarly with other TeNT-resistant VAMP, and adjust, when appropriate, the operating conditions.

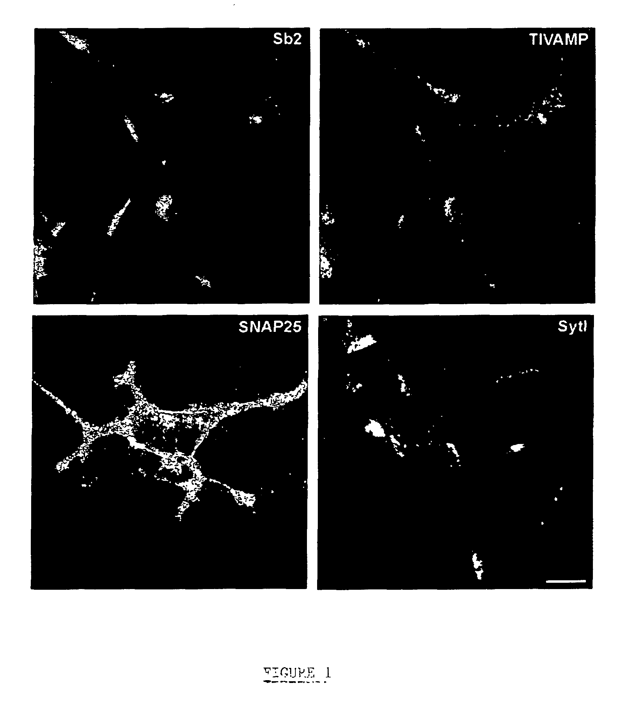

**Figure 1** illustrates the localization of membrane markers in horizontal confocal sections of staurosporine-differentiated PC12 cells.
PC12 cells were treated with 100nM staurosporine for 24 hours, fixed and processed for immunofluorescence with anti-synaptobrevin 2 (Sb2) and anti-TI-VAMP (TIVAMP), anti-SNAP25 (SNAP25) or anti-synaptotagmin I (Syt I) antibodies. The cells were then observed by confocal microscopy. Note the lack of colocalization of synaptobrevin 2 and TI-VAMP, the restricted localization of SNAP25 at the plasma membrane and the concentration of synaptotagmin I at the tip of neurites. Bar: 5µm.

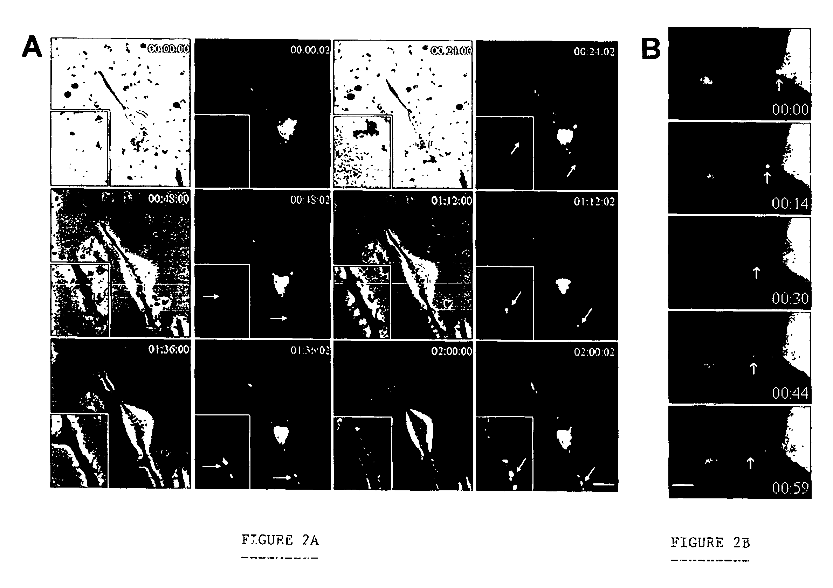

**Figures 2** illustrate the dynamics of GFP-TIVAMP-vesicles : PC12 cells transfected with GFP-TIVAMP were treated with staurosporine for 5 hours and observed under time-lapsed videomicroscopy in the presence of staurosporine.
**Figure 2A** illustrates that the GFP-TIVAMP vesicles accompany the growth of neurites. Transmission and fluorescent light images were recorded every 2 min over a period of 8 hours. Images recorded every 24 min through the middle period of the whole recording are shown. The inset shows a higher magnification of a growing neurite. Arrows indicate regions of this neurite where GFP-TIVAMP concentrates. Bar: 5µm.
**Figure 2B** illustrates the GFP-TIVAMP vesicle dynamics in neurites. Fluorescent light images were recorded every 15s over a period of 30 min (bottom right number: time in s). Images recorded during a 1min period of the recording are shown. The arrow indicates a GFP-TIVAMP vesicles which is moving anterogradly. Bar: 1 µm.

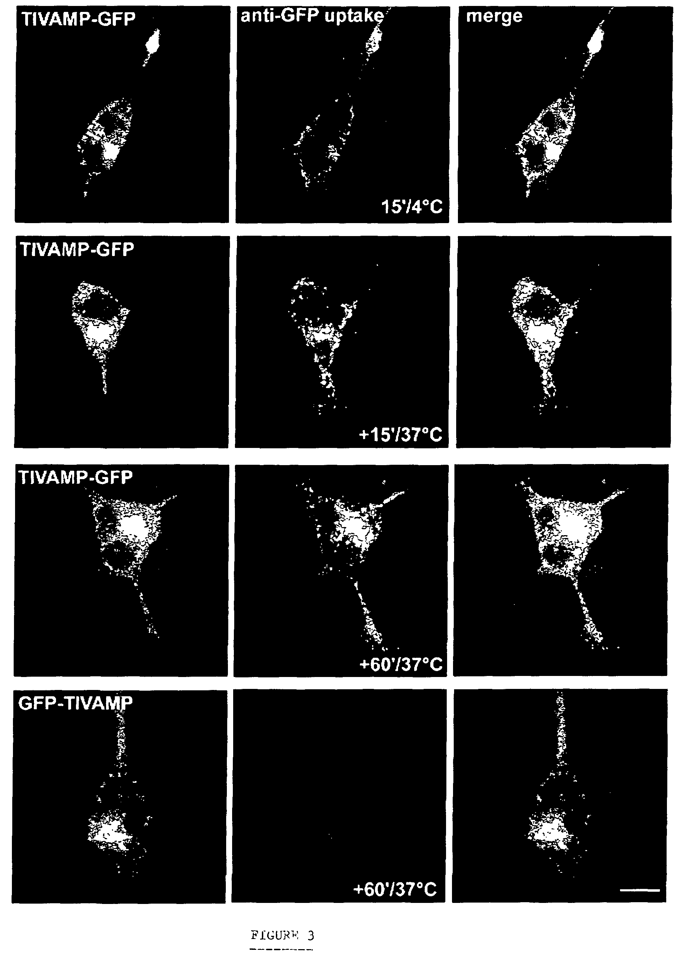

**Figure 3** illustrates TI-VAMP recycles at the neuritic plasma membrane. PC12 cells transfected with TIVAMP-GFP or GFP-TIVAMP and treated with staurosporine for 20 hours were placed on ice, incubated with monoclonal antibody anti-GFP (5µg/ml) for 15 min and directly fixed (15'/4°C) or further incubated at 37°C for 15 min (+15'/37°C) or 60 min (+60'/37°C) before fixation. Note the dense labelling of the neuritic plasma membrane in the 15'/4°C and +15'/37°C conditions. Full loading of the GFP-TIVAMP compartment is reached in the +60'/37°C condition. Bar: 5µm.

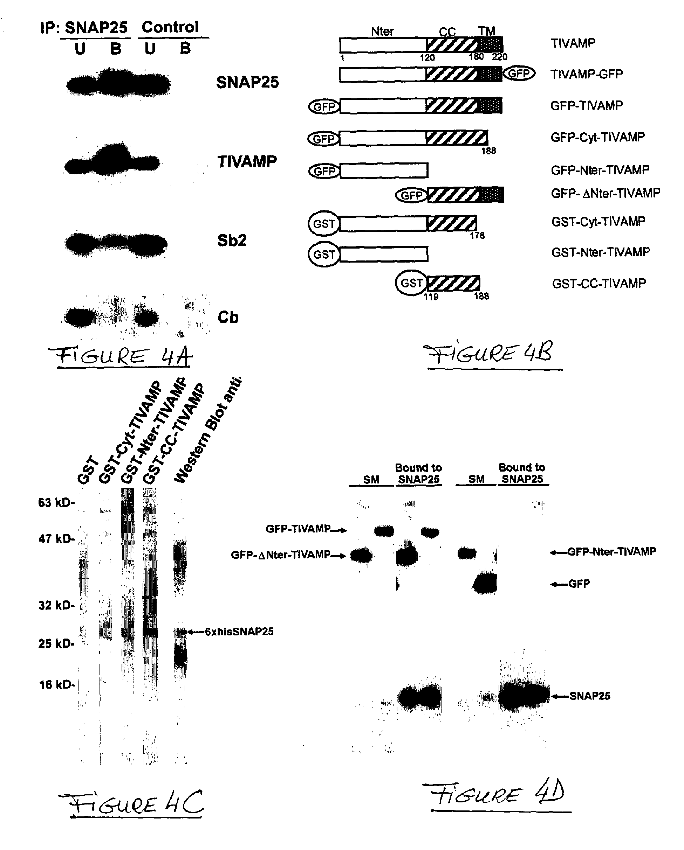

**Figures 4** illustrate the biochemical properties of the TI-VAMP/SNAP25 complex.
**Figure 4A** illustrates that TI-VAMP forms a complex with SNAP25 in Triton X100 extract of rat brain. Immunoprecipitation with anti-SNAP25 antibodies was performed from Triton X100 soluble extract of rat brain as described in the Materials and Methods section of example 1 and immunoprecipitated proteins were detected by western blot analysis with the indicated antibodies (synaptobrevin 2, Sb2; cellubrevin, Cb; U, unbound, B ; bound to anti-SNAP25 immunobeads). The bound fraction corresponded to a 65-fold enrichement compared to unbound. The SNAP25/TI-VAMP complex seemed more abundant than the SNAP25/synaptobrevin 2 one but this may only reflect a lower expression level of TI-VAMP compared to synaptobrevin 2 in the adult brain. Note that cellubrevin did not co-immunoprecipitate with SNAP25.
**Figure 4B** illustrates the structure of TI-VAMP and TIVAMP-derived constructs. TI-VAMP is composed of three domains: the Nter domain (aminoacids 1 to 120), the coiled-coiled domain, also called R-SNARE motif, (CC, amino acids 121 to 180), and one comprising the transmembrane domain and a short luminal domain (TM, amino acids 181 to 220). These domains were tagged with GFP and GST as depicted.
**Figure 4C** illustrates that the Nter domain of TI-VAMP inhibits binding of TI-VAMP to SNAP25.
The binding of GST, GST-Cyt-TIVAMP, GST-Nter-TIVAMP, or GST-CC-TIVAMP was measured by overlay over immobilized 6xhis-SNAP25 (indicated by the arrow). GST-CC-TIVAMP bound efficiently to immobilized 6xhis-SNAP25. Little binding of GST-Cyt-TIVAMP and none of GST and GST-Nter-TIVAMP were observed. As positive control, a strip was revealed with anti-6xhistidine antibodies.
**Figure 4D** illustrates that the TI-VAMP mutant lacking the Nter domain coimmunoprecipitates with SNAP25 more efficientely than full-length TI-VAMP. HeLa cells cotransfected with SNAP25 plus GFP-ΔNter-TIVAMP, GFP-TIVAMP, GFP-Nter-TIVAMP or GFP were lysed and subjected to immunoprecipitation with mouse monoclonal anti-SNAP25 antibodies as described in the Materials and Methods section of example 1. The immunoprecipitated proteins were then detected by western blot with anti-GFP or anti-SNAP25 rabbit polyclonal antibodies. The bound fraction corresponded to a 100-fold enrichment compared to the starting material (SM) in the case of the GFP blot and to a 10-fold enrichment in the case of the SNAP25 blot. Note that neither GFP-Nter-TIVAMP nor GFP coimmunoprecipitated with SNAP25.

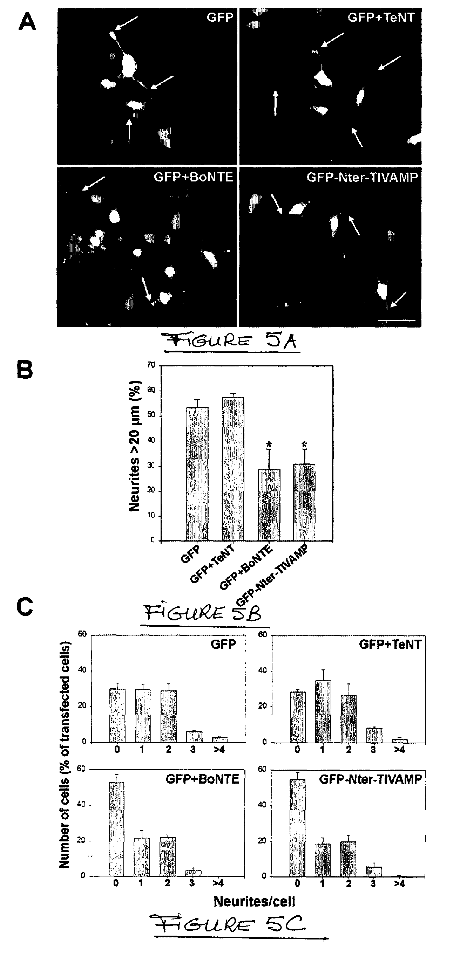

**Figures 5** illustrate the expression of the Nter domain of TI-VAMP inhibits neurite outgrowth.
**Figure 5A** illustrates the effect of GFP, GFP plus TeNT, GFP plus BoNT E or GFP-Nter-TIVAMP on neurite outgrowth. PC12 cells transfected with the indicated constructions and treated with staurosporine were fixed and direct fluorescence images were recorded. Representative fields of the distinct phenotypes found are shown. Note the long neurites displayed both by the GFP and the GFP+TeNT-transfected cells compared with the shorter ones displayed by the GFP+BoNTE and the GFP-Nter-TIVAMP-transfected cells (arrowheads). Bar: 25µm
**Figure 5B** illustrates that GFP-Nter -TIVAMP and BoNT E inhibit neurite length. Percentage of neurites longer than 20µm. A minimun of 50 transfected cells of each type were recorded in blind, and the length of all their neurites was measured. The mean values (+/- SE) of percentage of neurites longer than 20µm from three independent experiments are shown. *p<0.03 (Student's t-test). Note the lack of effect of TeNT and that BoNT E and GFP-Nter-TIVAMP had a similar inhibitory effect on neurite length.
**Figure 5C** illustrates the number of neurites per cell. The same randomly chosen transfected cells were use to quantify the number of neurites per cell. Shown is the number of cells, expressed as the percentage of transfected cells, displaying 1, 2, 3 or >4 neurites. The mean values (+/- SE) of three independent experiments are shown. Note the lack of effect of TeNT and that both BoNT E and GFP-Nter-TIVAMP enhanced the % of cells without neurites.

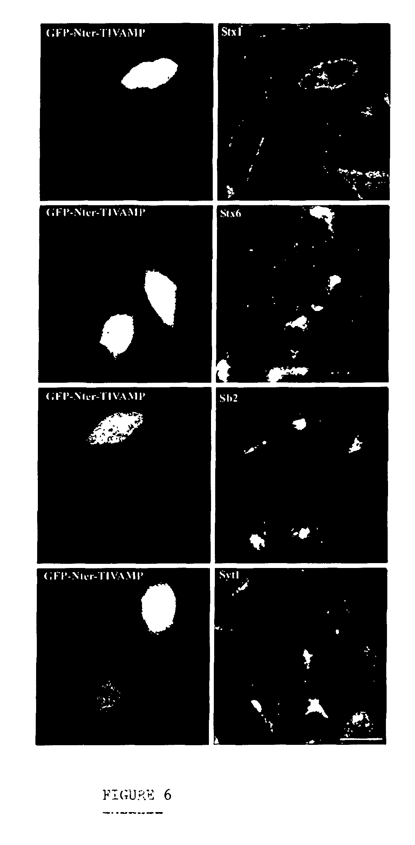

**Figure 6** illustrates the morphology of GFP-Nter-TIVAMP expressing cells. PC 12 cells transfected with GFP-Nter-TIVAMP and treated with staurosporine as in figure 5 were fixed, processed for double fluorescence by combining direct GFP fluorescence detection with indirect imunofluorescence detection using the indicated antibodies. Representative GFP-Nter-TIVAMP transfected cells without or with short neurite(s) are shown in horizontal confocal sections. Syntaxin (Stx) 1 and 6 and synaptobrevin 2 (Sb2) have a localization similar in untransfected as in GFP-Nter-TIVAMP expressing cells. Synaptotagmin I immunoreactivity was weaker in GFP-Nter-TIVAMP transfected cells than in untransfected cells. Bar: 10µm.

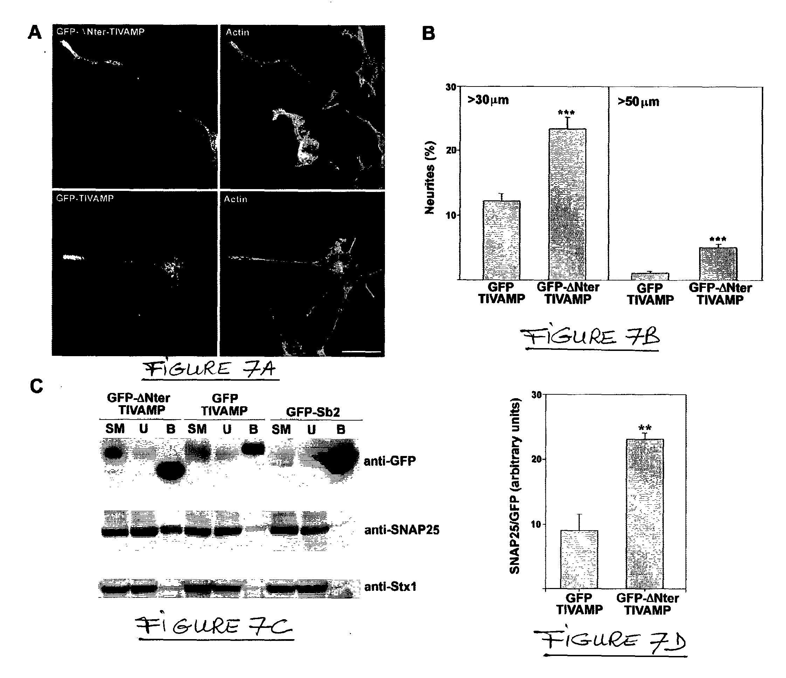

**Figures 7** illustrate that the expression of a "deleted" polypeptide of the invention (TI-VAMP lacking the Nterminal domain) enhances neurite outgrowth.
**Figure 7A** illustrates the morphology of PC12 cells transfected with GFP-TIVAMP or GFP-ΔNter-TI-VAMP. The cells were transfected, treated with staurosporine as in figure 5, fixed/permeabilized and processed for double fluorescence by combining direct GFP fluorescence detection with indirect imunofluorescence detection using Texas Red-phalloidin to visualize the actin filaments. Note the occurrence of numerous filopodia in the neuritic tip of the GFP-ΔNter-TI-VAMP transfected cell. Bar: 10µm
**Figure 7B** illustrates the GFP-ΔNter-TIVAMP increases neurite length. A minimun of 100 transfected cells of each type were recorded in blind, and the length of all their neurites was measured. The mean values (+/- SE) of the percentage of neurites longer than 30µm or 50µm from three independent experiments is shown. *** indicates p< 0.001 (Student's t-test).
**Figure 7C** illustrates the GFP-ΔNter-TIVAMP enhances formation of SNARE complexes. A Triton X-100 soluble extract was prepared from PC12 cells transfected with GFP-TIVAMP, GFP-ΔNter-TIVAMP or GFP-Sb2 and subjected to overnight immunoprecipitation with monoclonal anti-GFP antibodies. Immunoprecipitated proteins were resolved in SDS-PAGE followed by western blot analysis with the indicated antibodies. Note the increased co-immunoprecipitation of endogenous SNAP25 with GFP-ΔNter-TIVAMP compared with GFP-TIVAMP. The histogram in the right side shows the quantification of the amount of endogenous SNAP25 immunoprecipitated normalize to the amount of GFP-fusion protein immunoprecipitated from two independent experiments. ^{**}p<0.01 (Student's t-test).

**Figure 8** illustrates the complete human TI-VAMP aminoacid (SEQ ID N°6) and polypeptide (SEQ ID N°5) sequences.

### EXEMPLE 1 :

### Materials and Methods

**Antibodies and clones:** Rabbit serums (TG11 and TG16) directed against TIVAMP were purified by affinity chromatography in a column loaded with a GST-fusion protein of the coiled-coil domain of TI-VAMP (see below).

Mouse monoclonal antibodies directed against synaptobrevin 2 (clone 69.1, available from Max Planck Institute, Goettingen, FRG) , SNAP25 (clone 20, Transduction Labs., San Diego, CA), GFP (clone 7.1 and 13.1, Boehringer, Mannheim, FRG), syntaxin 6 (clone 30, Transduction Labs, San Diego, CA), syntaxin 1 (HPC-1, available from Yale University, New Haven, CT), glutathione S-transferase (generous gift from J.-L. Theillaud, Institut Curie, Paris, France), TeNT light chain (TeNT-LC) (generous gift from H. Niemann, Hannover Medical School, Hannover, FRG), rabbit polyclonal antibodies against the ectoplasmic domain of synaptotagmin I (8907, available from Yale University, New Haven, CT), SNAP25 (MC9, available from Yale University, New Haven, CT), GFP (Boehringer, Mannheim, FRG) and histidine (Santa Cruz Biotechnology Inc., Santa Cruz, CA) have been described previously. Affinity-purified Cy2 and Texas Red-coupled goat anti-mouse and anti-rabbit immunoglobulins were purchased from Jackson ImmunoResearch (West Grove, PA). Rhodamine-coupled phalloidin was from SIGMA (Saint-Louis, MO). Alkaline Phosphatase coupled-sheep anti-mouse were from Promega (Madison, WI). The cDNAs of human TI-VAMP and cellubrevin were previously described. The cDNA sources were : rat synaptobrevin 2 (R. Scheller, Stanford University, Stanford, CA), for rat SNAP25A (R. Jahn, Max Planck Institute, Goettingen, FRG), for TeNT-LC and BoNT-LC (H. Niemann, Hannover Medical School, Hannover, FRG) and for ratiometric pHLuorin (G. Miesenbock, Sloan Kettering Memorial Hospital, New York, NY).

**Cell culture:** PC12 cells were cultured in RPMI supplemented with 10% horse serum (HS) and 5% fetal calf serum (FCS) under standard conditions for PC12 cells. Cells were plated either on collagen-coated plastic dishes or on poly(L)lysine plus collagen-coated glass coverslips. HeLa cells were cultured in DMEM supplemented with 10% FCS.

**DNA constructions:** For production of N-terminal-GFP-fusion proteins the distinct cDNAs were cloned into the pEGFP-C3 vector (Clontech, Palo Alto, CA). The same empty vector was also used as a control in some of the neurite outgrowth assays. Full-length TIVAMP (TIVAMP ; aa : SEQ ID N°6 ; DNA : SEQ ID N°5), Nterminal domain-TIVAMP (Nter-TIVAMP, from M¹ to N¹²⁰; aa : SEQ ID N°2 ; DNA : SEQ ID N°1), cytosolic domain-TIVAMP (Cyt-TIVAMP, from M¹ to K¹⁸⁸ ; aa : SEQ ID N°8 ; DNA : SEQ ID N°7), □Nterminal domain-TIVAMP (□Nter-TIVAMP, from M¹⁰² to the end ; aa : SEQ ID N°4 ; DNA : SEQ ID N°3), and full-length synaptobrevin 2 (Sb2), were obtained by PCR using standard procedures and the following sets of oligonucleotides:
5'-ATGGCGATTCTTTTTGCTGTTGTTGCC-3' (SEQ ID N°9) and 5'CTATTTCTTCACACAGCTTGGCCATGT-3' (SEQ ID N° 10) for TI-VAMP; 5'-ATGGCGATTCTTTTTGCTGTTGTTGCC-3' (SEQ ID N°11) and 5'-CTTATTCTCAGAGTGATGCTTCAGCTG-3' (SEQ ID N°12) for Nter-TI-VAMP; 5'-ATGGCGATTCTTTTTGCTGTTGTTGCC-3' (SEQ ID N°13) and 5'-ATCCTACTTGAGGTTCTTCATACACATGGCTC (SEQ ID N°14) for Cyt-TI-VAMP; 5'-ATGAATAGCGAGTTCTCAAGTGTCTTA-3' (SEQ ID N°15) and 5'-CTATTTCTTCACACAGCTTGGCCATGT-3' (SEQ ID N°16) for □Nter-TI-VAMP; 5'-ATGTCGGCTACCGCTGCCACCGTCCCG-3' (SEQ ID N°17) and 5'-TTAAGAGCTGAAGTAAACTATGATGAT for Sb.2 (SEQ ID N°18). TI-VAMP, Nter-TI-VAMP, Cyt-TI-VAMP and Cyt-Syb2 were cloned in pEGFP-C3 using the KpnI/XbaI sites, while □Nter-TI-VAMP was cloned in HindIII/XbaI sites.

On figure 8, is shown the complete human TI-VAMP sequences SEQ ID N°5 (DNA) and SEQ ID N°6 (aminoacids; from M¹ to K²²⁰).

Nter aminoacid sequence corresponds to human TI-VAMP sequence from M¹ to N¹²⁰ (SEQ ID N°2). Nter DNA sequence bears SEQ ID N° 1.

ΔNter-TI-VAMP aminoacid sequence corresponds to human TI-VAMP sequence from M¹²⁰ to K²²⁰ (SEQ ID N°4). ΔNter-TI-VAMP DNA sequence bears SEQ ID N°3.

Cytosolic TI-VAMP aminoacid sequence corresponds to human TI-VAMP sequence from M¹ to K¹⁸⁸ (SEQ ID N°8). Cyt-TI-VAMP DNA sequence bears SEQ ID N°7.

The 101 aminoacid fragment which lacks from ΔNter-TI-VAMP by comparison with the complete human TI-VAMP sequence, i.e. the M¹-A¹⁰¹ fragment, bears SEQ ID N°20. Its corresponding DNA sequence bears SEQ ID N° 19.

For production of the C-terminal-GFP (ratiometic pHLuorin in this case) fusion protein of TIVAMP, TIVAMP cDNA bearing a BamHI site in its 3' was obtained by PCR using the 5'-GGATCCTTTCTTCACACAGCTTGGCCA-3' (SEQ ID N°21) and 5'-CTATTTCTTCACACAGCTTGGCCATGT-3' (SEQ ID N°22) oligonucleotides, and cloned in the pCR3.1-Uni vector (Clontech, Palo Alto, CA). Ratiometric pHLuorin was then cloned in the BamHI/EcoRI sites. Nter-TIVAMP, Cyt-TIVAMP, and coiled-coiled domain of TIVAMP (CC-TIVAMP; from E¹¹⁹ to K¹⁸⁸) were fused to glutathione S-tranferase (GST) gene by cloning in pGEX4T vector (Pharmacia, Saclay, France).

**Overlay assay:** The corresponding GST fusion proteins and GST alone were produced and purified as previously described. 6xhis-tagged SNAP25A (6xhisSNAP25, bacterial strain is available from G. Schiavo, ICRF, London, UK) was purified.

6xhisSNAP25 was run on SDS-PAGE and western blotted onto Immobilon-P membrane (Millipore, Bedford, MA). The amount of 6xhisSNAP25 corresponds to 1.25µg/mm of membrane. 4mm strips of the membrane were cut and incubated in 150mM NaCl, 5% non fat dry milk, 50mM phosphate pH7.5 buffer for 1 hour at room temperature. The strips were then incubated with 10nM the GST-fusion proteins overnight at 4°C in buffer B (3% BSA, 0.1% Tween 20, 20mM Tris pH: 7.5) containing 1 mM DTT. The strips were rinsed three times in buffer B at room temperature, incubated with anti-GST antibodies in buffer B for 1 hour, rinsed in buffer B three times and incubated with alkaline phosphatase-coupled sheep anti-mouse antibodies. The detection was carried out simultaneously for all the strips, for the same time, using a kit from Promega (Madison, WI).

**Cell transfection:** PC12 or HeLa cells were trypsinized, washed and resuspended at a density of 7.5-10 x 10⁶ cells/ml in Optimix (Equibio, Boughton Monchelsea, UK). Electroporation was performed with 10µg DNA in a final volume of 0.8ml cell suspension using a Gene Pulser II device (Bio-Rad, Hercules, CA) with one shock at 950µF and 250 V. When GFP was cotransfected with TeNT or BoNTE for monitoring the transfected cells, the plasmid carrying the GFP gene was added at double concentration in order to ensure that all the cells that uptake it do also uptake the plasmid carrying the toxin. Immediately after electroporation, cells were washed with 5ml of complete medium before plating them for immunoprecipitation or immunofluoresence microscopy analysis. Five hours later, the outgrowth medium was removed and replaced with fresh medium containing 100nM staurosporine (SIGMA, St Louis, MO). PC12 and HeLa cells were processed 24 or 48 hours after transfection respectively. For enhanced expression of the exogenous proteins, 5mM sodium butyrate was added in all the cases during the last 6 hours before processing the cells.

**Antibody uptake assay:** PC12 cells processed as indicated above were incubated in the presence of 5µg/ml anti-GFP antibody in culture medium for 15min on ice, 15min on ice then 15min at 37°C, or 15min on ice then 60min at 37°C, 24 hours after transfection with GFP-TIVAMP or TIVAMP-GFP. The cells were then washed twice with culture medium and twice with PBS, fixed with PFA and processed for immunofluorescence.

**Immunocytochemistry:** Cells were fixed with 4% PFA and processed for immunofluorescence. Optical conventional microscopy was performed on a Leica microscope equipped with a MicroMax CCD camera (Princeton Instruments, Princeton, NJ). Confocal laser scanning microscopy was performed using a TCS confocal microscope (Leica, Heidelberg, FRG). Images were assembled without modification using Adobe Photoshop (Adobe Systems, San Jose, CA).

**Neurite outgrowth assay:** Cells were fixed 24 hours after transfection. Between 20 and 100 randomly chosen fields for each condition were taken with a MicroMax CCD camera (Princeton Instruments, Princeton, NJ), resulting in the analysis of at least 50 transfected cells. A neurite was defined as a thin process longer than 5µm. Using the Metamorph software (Princeton Instruments, Princeton, NJ) two parameters were scored in each case: the number of neurites per cell (from 0 to 4 or more neurites), and the length of each neurite, from the cell body limit until the tip of the process. The obtained data were analysed for their statistical significance with SigmaStat (SPSS Inc., Chicago, IL). All the recordings and the Metamorph analysis were done in blind.

**Videomicroscopy:** Living PC12 cells transfected and treated with staurosporine as described above were placed in complete medium in an appropriate chamber equilibrated at 37°C and 5% CO₂. Cells were monitored with a MicroMax CCD camera (Princeton Instruments, Princeton, NJ) for as much as 9 hours, taking images both through phase contrast and FITC fluorescence every 2 min or every 15 seconds. Images were assembled using Metamorph (Princeton Instruments, Princeton, NJ).

**Immunoprecipitation:** Immunoprecipitation from rat brain was performed using a Triton X-100 soluble membrane fraction prepared as follows: two adult rat brains were homogenized with a glass/teflon homogenizer (9 strokes at 900 rpm) in 25 ml of 0.32M sucrose containing a protease inhibitor cocktail. All the steps were carried out at 4°C. After 10 min centrifugation at 800g the supernatant was centrifuged at 184000g for 1 hour, obtaining a cytosolic and a membrane fraction in the supernatant and the pellet, respectively. The pellet was resuspended in TSE (50mM Tris pH 8.0, 0.5mM EDTA, 150mM NaCl) containing 1% Triton X-100 for 30 min and finally the insoluble material was removed by centrifugation at 184000g for 1 hour. Immunoprecipitation with anti-SNAP25 antibodies and mouse control IgGs was performed from 2mg of proteins from the soluble extract. Immunoprecipitations from transfected PC12 or Hela cells were performed using a total Triton X-100 soluble fraction prepared as follows: after two washes with cold TSE, cells were lysed for 1 hour under continuous shaking with TSE containing 1%Triton X-100 and protease inhibitors. The supernatant resulting from centrifugation at 20000g for 30 min was used for immunoprecitation. After overnight incubation of the brain and cell extracts with the antibodies, 50 µl of magnetic beads (Dynabeads, Dynal A.S., Oslo, Norway) were added for 2-4 hours. The magnetic beads were washed four times with TSE containing 1%Triton X-100, eluted with gel sample buffer and the eluates were boiled for 5 min and run on SDS-PAGE gels.

□Nter-TI-VAMP aminoacid sequence corresponds to human TI-VAMP sequence from M¹²⁰ to K²²⁰ (SEQ ID N°3). □Nter-TI-VAMP DNA sequence bears SEQ ID N°4.

Cytosolic TI-VAMP aminoacid sequence corresponds to human TI-VAMP sequence from M¹ to K¹⁸⁸ (SEQ ID N°7). Nter DNA sequence bears SEQ ID N°8.

The 101 aminoacid fragment which lacks from □Nter-TI-VAMP by comparison with the complete human TI-VAMP sequence, i.e. the M¹-A¹⁰¹ fragment, bears SEQ ID N°19. Its corresponding DNA sequence bears SEQ ID N°20.

### Results

### TI-VAMP dynamics in staurosporine-treated PC12 cells

Differentiation of neurons and nerve growth factor (NGF)-induced neurite outgrowth of PC 12 cells take several days. On the contrary, staurosporine, a protein kinase inhibitor, induces maximal neurite outgrowth in 24 hours of treatment in PC12 cells. Our neurite outgrowth assay is based on treating PC12 cells with 100 nM staurosporine for 24 hours. These experimental conditions do not induce apoptosis in PC12 cells. Figure 1 shows that synaptobrevin 2, TI-VAMP, SNAP25 and synaptotagmin I had a normal subcellular localization in staurosporine-treated PC12 cells. Synaptobrevin 2 concentrated in the perinuclear region and in neuritic tips. TI-VAMP positive vesicles were scattered throughout the cytoplasm and concentrated at the leading edge of extending neurites. Synaptotagmin I appeared almost exclusively in neurites and varicosities and SNAP25 was present throughout the plasma membrane. This pattern of immunostaining was similar to that observed in NGF-treated PC 12 cells, demonstrating the validity of this cellular model to study neurite outgrowth.

We produced TI-VAMP carrying a Green Fluorescent Protein (GFP) tag fused to the N-terminal end (GFP-TIVAMP, Figure 4B). Upon transfection of this construct in PC12 cells, GFP staining was indistinguishable from that of endogenous TI-VAMP (compare figure 2A with figure 1), thus discarding the possibility that fusion of the GFP tag could alter TI-VAMP trafficking. We then observed TI-VAMP dynamics by time lapsed video-microscopy in staurosporine-treated PC12 cells, which had been previously transfected with GFP-TIVAMP (figure 2). Fast growing neurites were recorded every 2 min over periods of 3 to 9 hours, 5 hours after the onset of staurosporine treatment. Figure 2A displays transmission and fluorescent light images recorded every 24 min during 2 hrs 02 min (see also accompanying movie). High magnification view of a neurite growing towards the bottom right of the image is shown in the inset. At each time point, GFP-TIVAMP containing vesicles distributed along this growing process, up to the leading edge of the growth cone (Figure 2A). Most movements of GFP-TIVAMP containing membranes were anterograde (Figure 2B).

We then constructed another form of fluorescent TI-VAMP by introducing a GFP tag at the C-terminus (TIVAMP-GFP, Figure 4B). In this case, the GFP tag is exposed to the extracellular medium following exocytosis of TI-VAMP containing vesicles. TIVAMP-GFP transfected PC12 cells were labelled with monoclonal antibodies directed against GFP while they were placed on ice, before fixation. The labelling was often concentrated at the tip of the growing neurite (Figure 3). When the cells were allowed to internalise the antibody at 37°C, we observed a fast, time-dependent uptake. After 15 min at 37°C, the anti-GFP immunoreactivity was seen in peripheral structures, very close to the plasma membrane with a low degree of overlap with the green signal emitted by the bulk of TIVAMP-GFP. After 60 min, most of the immunoreactvity co-localized with TIVAMP-GFP, indicating that the anti-GFP antibody had reached the entire TIVAMP-GFP compartment. We did not detect any plasma membrane labelling nor GFP antibody internalisation in GFP-TIVAMP transfected or untransfected cells (Figure 3) thus demonstrating the lack of capture of the antibody by fluid phase uptake. Altogether, these studies demonstrate that the dynamics of TI-VAMP containing vesicles very closely accompanies the growth of neurites and that the protein recycles at the neuritic plasma membrane.

### The N-terminal domain of TI-VAMP inhibits SNARE complex formation

Because TI-VAMP resists to NT treatment, new experimental approaches had to be developed to study its function in living cells. Toward this goal, we searched for mutated forms of TI-VAMP that would have impaired SNARE complex formation activity. We first identified SNAP25 as a main physiological target SNARE (t-SNARE) of TI-VAMP. SNAP25, a neuronal plasma membrane Q-SNARE, formed abundant SNARE complexes with TI-VAMP as seen by co-immunoprecipitation experiments performed from brain extracts. Cellubrevin, a v-SNARE that is expressed in glial cells but not in neurons, did not associate with SNAP25 thus showing that the SNARE complexes were not formed during solubilisation of brain membranes (figure 4A).

Protein sequence analysis of TI-VAMP shows that the protein has an original N-terminal (Nter) domain of 120 amino acids, located upstream of the coiled-coiled domain (also called R-SNARE motif). This Nter domain includes three regions predicted to be α helical by Hydrophobic Cluster Analysis and Jpred. This is reminiscent of the Nter domain of syntaxin 1, which comprises 3 α helices and inhibits lipid bilayer fusion. The Nter domain of Ssolp, the yeast homologue of syntaxin 1, inhibits the rate of SNARE complex formation. Similar Nter domains are present in the other plasma membrane but not in intracellular syntaxins, indicating that this function may be specific for exocytosis. This led us to prepare the following GST-fusion proteins: full cytoplasmic domain of TI-VAMP (GST-Cyt-TIVAMP), coiled-coiled domain alone (GST-CC-TIVAMP) and Nter domain alone (GST-Nter-TIVAMP) (Figure 4B), and to measure the binding of the corresponding proteins to immobilized 6xhis-SNAP25 in an overlay assay. GST-CC-TIVAMP bound very efficiently immobilized his-SNAP25 whereas GST-Cyt-TIVAMP bound very poorly. As controls, GST alone and GST-Nter-TIVAMP did not bind immobilized his-SNAP25 (Figure 4C). HeLa cells do not express endogenous SNAP25 so, we used them to study the association of SNAP25 with GFP-TIVAMP, GFP-□Nter-TIVAMP, GFP-Nter-TIVAMP (Figure 4B) or GFP, *in vivo,* following co-transfection. We measured the amount of GFP-tagged proteins co-immunoprecipitating with SNAP25 from Triton X-100 soluble extracts. GFP-□Nter-TIVAMP formed more abundant SNAP25-containing SNARE complexes than GFP-TIVAMP. As controls, GFP and GFP-Nter-TIVAMP did not bind SNAP25 (figure 4D). Altogether, we propose that the Nter domain exerts an intramolecular inhibition of the SNARE complex formation activity of TI-VAMP's coiled-coiled domain.

### TI-VAMP mediates neurite outgrowth

An assay was set up to measure the effect of transfection of NTs and TI-VAMP mutants on staurosporine-induced neurite outgrowth in PC12 cells. First, we showed that when cells were electroporated with two plasmids, virtually all cells expressed both transgenes. This was demonstrated by transfection with GFP-cellubrevin (GFP-Cb) alone, TeNT alone, or both. Co-transfection of TeNT with GFP-Cb resulted in total proteolysis of GFP-Cb. Second, the activity of transfected TeNT and BoNT E were demonstrated by complete proteolysis of endogenous synaptobrevin 2 and SNAP25 respectively.

In a first set of experiments, PC12 cells were transfected with GFP alone, GFP plus TeNT, GFP plus BoNT E or the Nter domain of TI-VAMP fused to GFP (GFP-Nter-TIVAMP, Figure 4B). The cells were then treated with staurosporine, and fixed after 24hours. Figure 5A shows a representative field observed in each condition. Neurites from cells transfected with GFP or GFP plus TeNT were similar to neurites from untransfected cells. Neurites from cells transfected with GFP plus BoNT E or GFP-Nter-TIVAMP were fewer and shorter. The length of neurites and the number of neurites per cell were measured in each GFP-positive cell, in each condition. GFP plus TeNT had no effect on neurite number and length compared to GFP alone. BoNT E reduced by 45% the number of neurites longer than 20 µm and strongly increased the number of cells without neurites (figure 5B,C). Expression of the Nter domain of TI-VAMP had an effect which was similar to that of BoNT E. GFP-Nter-TIVAMP reduced by 42% the number of neurites longer than 20µm and strongly increased the number of cells without neurites (figure 5B,C). The effects of GFP plus BoNT E and GFP-Nter-TIVAMP were statistically different from GFP alone with p= 0.027 and 0.017 (Student's t-test) respectively. The effects of BoNT E and GFP-Nter-TIVAMP were not additive, indicating that they act on the same exocytotic mechanism. In a different set of experiments, we measured the effect of GFP and GFP-Cyt-TIVAMP. GFP-Cyt-TIVAMP (neurites longer than 20µm: 50.2%+/-0.25) had no effect on neurite length compared to GFP (neurites longer than 20 µm: 50.7%+/-3.5). GFP-Cyt-TIVAMP had no effect on the number of neurites per cell. These results demonstrated that neurite outgrowth in staurosporine-treated cells is insensitive to TeNT but sensitive to BoNT E as in neurons. The fact that GFP-Nter-TIVAMP inhibited neurite outgrowth as strongly as BoNT E suggests that TI-VAMP plays a major role in neurite outgrowth.

We then checked that GFP-Nter-TIVAMP expression did not have a deleterious effect. Figure 6 shows a gallery of double immunofluorescence experiments performed in GFP-Nter-TIVAMP transfected cells. We observed no effect on the localization of syntaxin 1, a plasma membrane SNARE, syntaxin 6, a Golgi apparatus SNARE (figure 6), and SNAP25 when compared to untransfected or GFP-transfected cells. Synaptobrevin 2 appeared both in the perinuclear region and in the shorter neurites emerging from GFP-Nter-TIVAMP cells (figure 6 and compare to figure 1). These cells showed a lower level of expression of synaptotagmin I. Synaptotagmin I was the vesicular marker which was the most enriched in the tip of the neurites in untransfected cells (Figure 1 and 6) so our result may suggest that synaptotagmin I reached the neuritic tip by a TI-VAMP dependent pathway. These results showed that the Nter domain of TI-VAMP had a specific inhibitory effect on neurite outgrowth.

We then tested the effect of GFP- ΔNter-TIVAMP expression and compared it with that of GFP-TIVAMP on neurite outgrowth. We observed the occurrence of unusually long neurites with an increased number of filopodia. Staining of actin filaments with fluorescent phalloidin showed that the neurites of GFP□ □Nter-TIVAMP-transfected cells showed cortical actin localization similar to GFP-TIVAMP-transfected cells (Figure 7A). The pattern of staining of tubulin, synaptobrevin 2, synaptotagmin 1, SNAP25 and syntaxin 1 was the same in GFP- ΔNter-TIVAMP as in GFP-TIVAMP transfected and in untransfected cells. The effect of GFP- ΔNter-TIVAMP was quantified as in the case of GFP- ΔNter-TIVAMP. GFP- ΔNter-TIVAMP expression doubled the number of neurites longer than 30 µm and multiplied by 5 the number of neurites longer than 50 µm when compared to the expression of GFP-TIVAMP (Figure 7B). GFP-TIVAMP had no effect on neurite length and number per cell compared to GFP alone. We observed no effect of GFP- ΔNter-TIVAMP on the number of neurites per cell. We checked that GFP- ΔNter-TIVAMP formed more abundant SNARE complexes with endogenous SNAP25 by measuring the amount of SNAP25 and syntaxin 1 which was co-immunoprecipitated with GFP-ΔNter-TIVAMP, GFP-TIVAMP and GFP-Syb2. GFP- ΔNter-TIVAMP /SNAP25 complex was 2.5 times more abundant than GFP-TIVAMP/SNAP25. Accordingly, GFP-ΔNter-TIVAMP co-immunoprecipitated more syntaxin 1 than GFP-TIVAMP (Figure 7C). These results showed that a form of TI-VAMP, which had a higher SNARE complex formation activity strongly, enhanced neurite outgrowth.

### Discussion

Tetanus neurotoxin Insensitive-Vesicle Associated Membrane Protein (TI-VAMP : DNA sequence SEQ ID N°5 ; aa sequence SEQ ID N°6) is a V-SNARE (vesicle-associated soluble N-ethylmaleide-sensitive fusion protein attachment receptor) which is known to be involved in transport to the apical plasma membrane in epithelial cells.

The present invention reports for the first time that a N-terminal fragment of a VAMP such as TI-VAMP can show biological functions *in vivo.*

It is herein demonstrated that a fragment corresponding to the N-terminal domain which precedes the SNARE motif of TI-VAMP plays an inhibitory role on the activity of the VAMP TI-VAMP, that SNAP25 is a target SNARE (t-SNARE) for TI-VAMP in cells such as neuronal cells (i.e. they form complexes in such cells), and that such a N-terminal fragment is capable of inhibiting the association of TI-VAMP with its target SNARE (SNAP25 in neuronal cells, SNAP23 in epithelial cells). The first 120 N-terminal aa fragment corresponds to SEQ ID N°2 (corresponding DNA sequence: SEQ ID N°1) ; the first 101 ones to SEQ ID N°20 (corresponding DNA sequence: SEQ ID N°19). Such N-terminal fragments are capable of inhibiting the membrane traffic activity of the cells into which they have been transfected : they inhibit their fusion functions, and in a particular aspect, their exocytic functions. Membrane traffic can be envisioned as a succession of vesicle budding, maturation, vectorial transport, tethering, docking, and lipid bilayer fusion events. Vesicular transport to and fusion at the plasma membrane, *i.e.* exocytosis, is responsible for the release of soluble compounds such as neurotransmitters in the extracellular medium and for surface expression of plasma membrane proteins and lipids. On a more mecanistic point of view, such Nter fragments are herein shown to be capable of inhibiting the formation of complexes involving VAMP, and notably complexes involving a VAMP such as TI-VAMP and at least one of its t-SNARE (SNAP 25, SNAP23, syntaxin1, syntaxin3). In fact, such N-terminal fragments may inhibit any cell function which involves a tetanus neurotoxin-resistant pathway (TeNT-resistant). The overwhole resulting effect of such N-terminal fragments on the properties of said cells is to inhibit their membrane traffic activity, *i.e.* the transport of components to its plasma membrane, notably through vesicular transport. Conversely, the TI-VAMP fragments which are deleted from their N-terminal domain (in so far that their coiled coil motif activity is appropriately preserved, *i.e.* said □Nter fragments can still bind to at least one of their t-SNARE) are herein shown to exert stimulating effects on the membrane traffic activity of the cells into which they have been transfected: they are capable of stimulating their fusion functions, and in a particular aspect, their exocytic functions. Conversely to Nter fragments, such □Nter fragments are herein shown to be capable of stimulating the formation of complexes involving VAMP, and notably complexes involving a VAMP such as TI-VAMP and at least one of its t-SNARE (SNAP 25, SNAP23, syntaxin1, syntaxin3). In fact, such □Nter fragments may stimulate any cell function which involves a tetanus neurotoxin-resistant pathway (TeNT-resistant). The overwhole resulting effect of such □Nter fragments on the properties of said cells is to inhibit their membrane traffic activity, *i.e.* the transport of components to its plasma membrane, notably through vesicular transport.
The invention thus offers new means for controlling membrane traffic into a cells, and in particular for regulating fusion functions into a cell such as exocytic functions, and notably vesicular transports of components to the plasma membrane. The means of the invention thus allow the regulation of very fundamental functions and properties of any cell that express a VAMP such as TI-VAMP. In this respect, the skilled persons can envisage and perform from the teaching of the invention a very wide range of applications, and indeed any applications involving the regulation of a traffic membrane and/or of a TeNT-resistant pathway. This notably includes the positive and negative control of neurite outgrowth and of cell motility, this latter application being of special interest in the case of metastasis-forming cells.

A particular aspect of the invention indeed more precisely relates to neurite outgrowth control: the present invention shows for the first time that SNARE-mediated vesicular transport is essential to neurite outgrowth, *i.e.* to axonal and dendritic maturation and differentiation. This is the first report of TI-VAMP mediating neurite outgrowth. Elongation of axon and dendrites, so-called neurite outgrowth, is a crucial event in neuronal differentiation and maturation ; and thus in the development of the nervous system. Membrane traffic in dendrites is also of importance for synaptic plasticity and memory.
It is herein further demonstrated that over-expression of a Nter fragment (such as SEQ ID N°2 or N°20) inhibits neurite outgrowth as strongly as botulinum neurotoxin (BoNT E), a neurotoxin which is known to abolish the expression of SNAP 25, the now-identified t-SNARE partner of TI-VAMP in cells such as neuronal cells. It is also observed that such Nter fragments inhibit the formation of TI-VAMP/SNAP25 complexes in neuronal cells, and that the reverse effects are induced by □Nter fragments of a VAMP such as TI-VAMP.

The above-described results indeed demonstrates that TI-VAMP-mediated vesicular transport is essential for neurite outgrowth. Expression of the N-terminal domain of TI-VAMP inhibits neurite outgrowth as strongly as BoNT E, which abolishes the expression of SNAP25, a plasma membrane SNARE partner of TI-VAMP. In the contrary, activation of neurite outgrowth and increased SNARE complex formation were observed when the N-terminus deletion mutant of TI-VAMP was expressed in PC12 cells.

A main conclusion from our work is that TI-VAMP is involved in neurite outgrowth in neuronal cells such as PC12 cells. Our findings that TI-VAMP interacts with SNAP25 in PC12 cells and in the brain is consistent with the involvement of SNAP25 in neurite outgrowth. The TI-VAMP-dependent vesicular transport mediating neurite outgrowth in PC12 cells likely corresponds to the outgrowth of axons and dendrites in developing neurons. Indeed, TI-VAMP concentrates in the leading edge of axonal and dendritic growth cones of hippocampal neurons in primary culture. In support of this conclusion, preliminary experiments have shown a decreased number of neurites in young hippocampal neurons, which were microinjected with anti-TIVAMP antibodies. Neurite outgrowth may be also very active in differentiated neurons because it may participate to post-synaptic morphological changes related to plasticity and learning. A role for SNAP25 in neuronal plasticity and learning has been proposed. Therefore, the TI-VAMP and SNAP25 dependent vesicular transport mechanism described here could also mediate activity-dependent exocytosis involved in dendrite elongation and post-synaptic receptor expression at the plasma membrane in mature neurons. This could account for the distribution of TI-VAMP containing vesicles throughout the dendrites and of SNAP25 in the dendritic plasma membrane of mature neurons. According to our present results, the proteic and lipidic map of TI-VAMP vesicular compartment is likely to identify factors, which are important for neurite elongation both in developing and mature neurons. The purification of TI-VAMP vesicular compartment allows to determine which other proteins are involved in this pathway, particularly rab proteins that have been shown to play a role in neurite outgrowth.

We found that the cytoplasmic domain of TI-VAMP, which comprises the N-terminal domain plus the R-SNARE motif, had no effect on neurite outgrowth whereas the N-terminal domain alone strongly inhibited it. This demonstrates that the full cytoplasmic domain is inactive *in vivo.* The coiled-coiled domain of TI-VAMP bound more efficiently SNAP25 than the cytoplasmic domain by overlay assay. Therefore, our observations favour a model in which the N-terminal domain of TI-VAMP acted as an intramolecular inhibitor of the R-SNARE motif preventing it from forming SNARE complexes. Hence, dentifying the signal transduction pathway(s) and factors, able to activate TI-VAMP, will be of crucial importance to further understand how neurite outgrowth is controlled.

Finally, our finding that the N-terminal domain of TI-VAMP plays an important function in the control of neurite outgrowth, suggests that this protein is a potential target of pharmacological agents that could modulate the activity of TI-VAMP by releasing the inhibition of this domain. Such agents could be used to specifically activate TI-VAMP mediated exocytosis thus, stimulate neurite outgrowth. Once identified, such drugs could be used in the treatment of nerve traumatisms such as spinal cord injury.

### EXEMPLE 2 : SEQ ID N°2 or N°20 (Nter) inhibits the motility of tumoral cells

Cells such as tumoral cells or the MDCK cell line (Mardin Darby Canine Kidney) can be used for transfection as above-described in example 1 so as to make them express a Nter polypeptide (M¹ to N¹²⁰ of TI-VAMP, of the SEQ ID N°1 and N°2 invention, a "deleted" polypeptide of the invention (M¹⁰² to the end of human TI-VAMP, SEQ ID N°3 and N°4), or the complete TI-VAMP sequence (SEQ ID N°5 and N°6).

These transfected cells can thus be placed into contact with a migration inductor such as a growth factor (*e.g.* HGF -Hepatocyte Growth Factor- for MKCK), and the differences in cell migration for each treatment can be observed video-microscopy and/or confocal microscopy.

When appropriate, or desired, *in vivo* observations can be performed.

### EXEMPLE3:

Appropriate formulations for drugs of the invention notably comprise tablet and injection solution, spray for chemical or peptidic products, and comprise liposome and virus for DNA products.

## Claims

1. Isolated polypeptide of which sequence corresponds to a sequence is chosen among the group consisting of a sequence corresponding to SEQ ID N°2, a sequence corresponding to SEQ ID N°20, the sequences corresponding to any conservative fragment of SEQ ID N°2, the sequences corresponding to any conservative fragment of SEQ ID N°20, the sequences corresponding to any conservative variant of SEQ ID N°2, the sequences corresponding to any conservative variant of SEQ ID N°20.

2. Isolated polypeptide of which sequence corresponds to a sequence chosen among the group consisting of the SEQ ID N°6 sequence of which N-terminal domain has been deleted from at least one polypeptide according to claim 1.

3. Isolated polypeptide of which sequence corresponds to a sequence chosen among the group consisting of SEQ ID N°4, the sequences corresponding to any conservative variant of SEQ ID N°4, and the sequences corresponding to any conservative fragment of SEQ ID N°4.

4. Product chosen among the group consisting of the monoclonal antibodies capable of binding to a polypeptide according to claim 1, and the Fab, F(ab')₂, CDS fragments thereof.

5. Product according to claim 4, **characterized in that** it is capable under physiological conditions of inhibiting at least one of the biological properties a polypeptide according to claim 1 can show.

6. Isolated polynucleotide of which sequence corresponds to a sequence coding for a polypeptide according to claim 1.

7. Isolated polynucleotide of which sequence corresponds to a sequence chosen among the group consisting of a sequence corresponding to SEQ ID N°1, a sequence corresponding to SEQ ID N°19, the sequences corresponding to any conservative fragment of SEQ ID N°1, the sequences corresponding to any conservative variant of SEQ ID N°1, the sequences corresponding to any conservative fragment of SEQ ID N°19, the sequences corresponding to any conservative variant of SEQ ID N°19.

8. Isolated polynucleotide of which sequence corresponds to a sequence coding for a polypeptide according to any one of claims 2-3.

9. Isolated polynucleotide of which sequence corresponds to a sequence chosen among the group consisting of the SEQ ID N°5 sequences of which 5' domain has been deleted from at least one polynucleotide according to any one of claims 6-7.

10. Isolated polynucleotide of which sequence corresponds to a sequence chosen among the group consisting of a sequence corresponding to SEQ ID N°3, the sequences corresponding to any conservative fragment of SEQ ID N°3, the sequences corresponding to any conservative variant of SEQ ID N°3.

11. Isolated polynucleotide of which sequence corresponds to a sequence coding for a product according to any one of claims 4-5.

12. Transfection vector, which comprises at least one polynucleotide according to claim 6 or 7.

13. Transfection vector, which comprises at least one polynucleotide according to any one of claims 8-11.

14. Genetically engineered cell comprising at least one element chosen among the group consisting of the polynucleotides according to claim 6, the polynucleotides according to claim 7, the transfection vectors according to claim 12.

15. Genetically engineered cell comprising at least one element chosen among the group consisting of the polynucleotides according to claims 8-11, the transfection vectors according to claim 13.

16. Pair of oligonucleotides **characterized in that** it is capable under standard PCR conditions to amplify at least one polynucleotide according to claim 6 or 7.

17. Pair of oligonucleotide of which respective sequences correspond to SEQ ID N°11 and SEQ ID N°12.

18. Pair of oligonucleotides **characterized in that** it is capable under standard PCR conditions to amplify at least one polynucleotide according to any one of claims 8-10.

19. Pair of oligonucleotides of which respective sequences correspond to SEQ ID N°15 and SEQ ID N° 16.

20. Isolated polynucleotide such as obtained by using on a polynucleotide population at least one pair of oligonucleotides according to claim 16 or 17.

21. Isolated polynucleotide such as obtained by using on a polynucleotide population at least one one pair of oligonucleotides according to claim 18 or 19.

22. Pharmaceutical composition comprising at least one product chosen among the group consisting of the polypeptides according to claim 1, the polynucleotides according to claims 6, 7, 20, the transfection vectors according to claim 12, and the cells according to claim 14.

23. Pharmaceutical composition comprising at least one product chosen among the group consisting of the polypeptides according to claims 2-3, the polynucleotides according to claims 8-11, 21, the transfection vectors according to claim 13, and the cells according to claim 15.

24. Method for identifying a pharmaceutical agent capable of stimulating a cell function chosen among the group consisting of the cell functions involving a membrane traffic, the cell functions involving a TeNT-resistant pathway, the cell functions involving the formation of complexes with TI-VAMP, the cell functions involving at least one TI-VAMP, the cell functions involving a fusion, or an exocytic event, the cell functions involved in neurite outgrowth, in neuronal maturation, in neuronal differentiation, in memory abilility, and in learning capacity, **characterized in that** it comprises at least one step chosen the group consisting of:
- the identification of an agent that is capable under physiological conditions of blocking or diminishing the inhibition effect that is observed when the cytoplasm of a cell is placed into contact with at least one product chosen among the group consisting of the polypeptides according to claim 1, the polynucleotides according to claim 6, 7, 20, the transfection vectors according to claim 12, the cells according to claim 14, under conditions appropriate to cell membrane trafficking,
- the identification of an agent that is capable under physiological conditions to compete with a product according to any one of claims 4-5 for binding to at least one polypeptide according to claim 1,
- the identification of an agent that is capable under physiological conditions of stimulating the formation of complexes involving at least one polypeptide according to any one of claims 2-3, and at least one t-SNARE,
- the identification of an agent that is capable under physiological conditions of exerting an additional or synergic effect on the cell function observed when the cytoplasm of a cell is placed under conditions appropriate to cell membrane trafficking, into contact with at least one product chosen among the group consisting of the polypeptides according to claims 2-3, the products according to claims 4, 5, the polynucleotides according to claims 8-11, 21, the transfection vectors according to claim 13, the cells according to claim 15.

25. Method for identifying a pharmaceutical agent capable of inhibiting a cell function chosen among the group consisting of the cell functions involving a membrane traffic, the cell functions involving a TeNT-resistant pathway, the cell functions involving the formation of complexes with TI-VAMP, the cell functions involving at least one TI-VAMP, the cell functions involving a fusion, or an exocytic event, the cell functions involved in cell motility, the cell functions involved in the formation of metastasis, **characterized in that** it comprises at least one step chosen among the group consisting of:
- the identification of an agent that is capable under physiological conditions of stimulating the inhibition effect observed when the cytoplasm of a cell is placed under conditions appropriate to cell membrane trafficking, into contact with at least one product chosen among the group consisting of the polypeptides according to claim 1, the polynucleotides according to claim 6, 7, 20, the transfection vectors according to claim 12, the cells according to claim 14,
- the identification of an agent that is capable under physiological conditions of inhibiting the formation of complexes involving at least one polypeptide according to any one of claims 2-3, and at least one t-SNARE,
- the identification of an agent that is capable under physiological conditions of exerting an inhibitory effect on a cell function that is observed when the cytoplasm of a cell is placed into contact, under conditions appropriate to cell membrane trafficking, with at least one product chosen among the group consisting of the polypeptides according to claims 2-3, the products according to claims 4-5, the polynucleotides according to claims 8-11, 21, the transfection vectors according to claim 13, the cells according to claim 15,
- the identification of an agent that is capable under physiological conditions to lyse a product according to claim 5.

26. A pharmaceutical agent such as obtained by the method according to claim 24.

27. A pharmaceutical agent such as obtained by the method according to claim 25.

28. The use of at least one product chosen among the group consisting of the polypeptides according to claim 1, the polynucleotides according to claim 6, 7,20 the transfection vectors according to claim 12, the cells according to claim 14, the pharmaceutical agents according to claim 27, for the production of a drug intended for at least one effect chosen among the group consisting of inhibiting a cell membrane traffic, inhibiting a vesicular transport, inhibiting a function involving a TeNT-resistant pathway, inhibiting a function involving at least one TI-VAMP, inhibiting the formation of complexes involving at least one TI-VAMP and at least one t-SNARE, inhibiting a cell motility, inhibiting the motility of cells susceptible of forming metastasis.

29. The use of at least one product chosen among the group consisting of the polypeptides according to any ones of claims 2-3, the products according to any one of claims 4-5, the polynucleotides according to any one of claims 8-11, 21, the transfection vectors according to claim 13, the cells according to claim 15, the pharmaceutical agents according to claim 26, for the production of a drug intended for at least one effect chosen among the group consisting of stimulating a cell membrane traffic, stimulating a vesicular transport, stimulating a function involving a TeNT-resistant pathway, stimulating a function involving at least one TI-VAMP, stimulating the formation of complexes involving at least one TI-VAMP and at least one t-SNARE, stimulating axonal and/or dendritic outgrowth, stimulating neuronal maturation, stimulating neuronal differentiation, stimulating memory and/or learning capacity, curing and/or palliating and/or preventing spinal cord trauma, curing and/or palliating and/or preventing neuro-degenerative disorders, curing and/or palliating and/or preventing sclerosis.

30. A method for diagnosing an undesired state, and/or for assessing the efficiency of a medical treatment, and/or for assessing the evolution of an undesired state, **characterized in that** it comprises at least one step chosen among the group consisting of:
- the detection in a biological sample of a presence significantly different from the standard level for at least one product chosen among the group consisting of the polypeptides according to claims 2-3, the products according to claims 4-5, the polynucleotides according to claims 8-11, 21, the transfection vectors according to claim 13, the cells according to claim 15,
- the detection in a biological sample of a level of expression for a polypeptide according to claim 1 significantly different from the standard level.

31. A method for identifying a compound capable of assuming the function of a biological effector of TI-VAMP, **characterized in that** it comprises:
- the detection of a compound which is capable under physiological conditions of binding to a polypeptide according to claim 1,
- the detection of a compound which is capable under physiological conditions of diminishing the inhibition effect that is observed when the cytoplasm of a cell expressing TI-VAMP is placed into contact with at least one product chosen among the group consisting of the polypeptides according to claim 1, the polynucleotides according to claim 6, 7, 20, the transfection vectors according to claim 12, the cells according to claim 14 under conditions appropriate to cell membrane trafficking,
- the detection of a compound which is capable under physiological conditions of exerting an additional or synergic effect on the cell function that is observed when the cytoplasm of a cell expressing TI-VAMP is placed into contact with at least one product chosen among the group consisting of the polypeptides according to claims 2-3, the products according to claims 4-5, the polynucleotides according to claims 8-11, 21, the transfection vectors according to claim 13, the cells according to claim 15, under conditions appropriate to cell membrane trafficking.


SEQUENCE LISTING

<110> Institut Curie
      C.N.R.S.

<120> CONTROL OF MEMBRANE TRAFFIC.

<130> 1100EP2000

<140>
<141>

<160> 22

<170> PatentIn Ver. 2.1

<210> 1
<211> 360
<212> DNA
<213> Homo sapiens

<400> 1
atggcgattc tttttgctgt tgttgccagg gggaccacta tccttgccaa acatgcttgg 60
tgtggaggaa acttcctgga ggtgacagag cagattctgg ctaagatacc ttctgaaaat 120
aacaaactaa cgtactcaca tggcaattat ttgtttcatt acatctgcca agacaggatt 180
gtatatcttt gtatcactga tgatgatttt gaacgttccc gagcctttaa ttttctgaat 240
gagataaaga agaggttcca gactacttac ggttcaagag cacagacagc acttccatat 300
gccatgaata gcgagttctc aagtgtctta gctgcacagc tgaagcatca ctctgagaat 360

<210> 2
<211> 120
<212> PRT
<213> Homo sapiens

<400> 2
Met Ala Ile Leu Phe Ala Val Val Ala Arg Gly Thr Thr Ile Leu Ala
  1               5                  10                  15

Lys His Ala Trp Cys Gly Gly Asn Phe Leu Glu Val Thr Glu Gln Ile
             20                  25                  30

Leu Ala Lys Ile Pro Ser Glu Asn Asn Lys Leu Thr Tyr Ser His Gly
         35                  40                  45

Asn Tyr Leu Phe His Tyr Ile Cys Gln Asp Arg Ile Val Tyr Leu Cys
     50                  55                  60

Ile Thr Asp Asp Asp Phe Glu Arg Ser Arg Ala Phe Asn Phe Leu Asn
65                  70                  75                  80

Glu Ile Lys Lys Arg Phe Gln Thr Thr Tyr Gly Ser Arg Ala Gln Thr
                85                  90                  95

Ala Leu Pro Tyr Ala Met Asn Ser Glu Phe Ser Ser Val Leu Ala Ala
            100                 105                 110

Gln Leu Lys His His Ser Glu Asn
        115                 120

<210> 3
<211> 357
<212> DNA
<213> Homo sapiens

<400> 3
atgaatagcg agttctcaag tgtcttagct gcacagctga agcatcactc tgagaataag 60
ggcctagaca aagtgatgga gactcaagcc caagtggatg aactgaaagg aatcatggtc 120
agaaacatag atctggtagc tcagcgagga gaaagattgg aattattgat tgacaaaaca 180
gaaaatcttg tggattcttc tgtcaccttc aaaactacca gcagaaatct tgctcgagcc 240
atgtgtatga agaacctcaa gctcactatt atcatcatca tcgtatcaat tgtgttcatc 300
tatatcattg tttcacctct ctgtggtgga tttacatggc caagctgtgt gaagaaa    357

<210> 4
<211> 119
<212> PRT
<213> Homo sapiens

<400> 4
Met Asn Ser Glu Phe Ser Ser Val Leu Ala Ala Gln Leu Lys His His
1               5                   10                  15

Ser Glu Asn Lys Gly Leu Asp Lys Val Met Glu Thr Gln Ala Gln Val
            20                  25                  30

Asp Glu Leu Lys Gly Ile Met Val Arg Asn Ile Asp Leu Val Ala Gln
        35                  40                  45

Arg Gly Glu Arg Leu Glu Leu Leu Ile Asp Lys Thr Glu Asn Leu Val
    50                  55                  60

Asp Ser Ser Val Thr Phe Lys Thr Thr Ser Arg Asn Leu Ala Arg Ala
65                  70                  75                  80

Met Cys Met Lys Asn Leu Lys Leu Thr Ile Ile Ile Ile Ile Val Ser
                85                  90                  95

Ile Val Phe Ile Tyr Ile Ile Val Ser Pro Leu Cys Gly Gly Phe Thr
            100                 105                 110

Trp Pro Ser Cys Val Lys Lys
        115

<210> 5
<211> 801
<212> DNA
<213> Homo sapiens

<400> 5
gggcctctag atgcatgctc gagcggccgc cagtgtgatg gatatctgca gaattcggct 60
tagactgaag ccatggcgat tctttttgct gttgttgcca gggggaccac tatccttgcc 120
aaacatgctt ggtgtggagg aaacttcctg gaggtgacag agcagattct ggctaagata 180
ccttctgaaa ataacaaact aacgtactca catggcaatt atttgtttca ttacatctgc 240
caagacagga ttgtatatct ttgtatcact gatgatgatt ttgaacgttc ccgagccttt 300
aattttctga atgagataaa gaagaggttc cagactactt acggttcaag agcacagaca 360
gcacttccat atgccatgaa tagcgagttc tcaagtgtct tagctgcaca gctgaagcat 420
cactctgaga ataagggcct agacaaagtg atggagactc aagcccaagt ggatgaactg 480
aaaggaatca tggtcagaaa catagatctg gtagctcagc gaggagaaag attggaatta 540
ttgattgaca aaacagaaaa tcttgtggat tcttctgtca ccttcaaaac taccagcaga 600
aatcttgctc gagccatgtg tatgaagaac ctcaagctca ctattatcat catcatcgta 660
tcaattgtgt tcatctatat cattgtttca cctctctgtg gtggatttac atggccaagc 720
tgtgtgaaga aatagaagcc gaattccagc acactggcgg ccgttactag tggatccgag 780
ctcggtacca agcttgatgc a                                           801

<210> 6
<211> 220
<212> PRT
<213> Homo sapiens

<400> 6
Met Ala Ile Leu Phe Ala Val Val Ala Arg Gly Thr Thr Ile Leu Ala
1               5                   10                  15

Lys His Ala Trp Cys Gly Gly Asn Phe Leu Glu Val Thr Glu Gln Ile
            20                  25                  30

Leu Ala Lys Ile Pro Ser Glu Asn Asn Lys Leu Thr Tyr Ser His Gly
        35                  40                  45

Asn Tyr Leu Phe His Tyr Ile Cys Gln Asp Arg Ile Val Tyr Leu Cys

    50                  55                  60

Ile Thr Asp Asp Asp Phe Glu Arg Ser Arg Ala Phe Asn Phe Leu Asn
65                  70                  75                  80

Glu Ile Lys Lys Arg Phe Gln Thr Thr Tyr Gly Ser Arg Ala Gln Thr
                85                  90                  95

Ala Leu Pro Tyr Ala Met Asn Ser Glu Phe Ser Ser Val Leu Ala Ala
            100                 105                 110

Gln Leu Lys His His Ser Glu Asn Lys Gly Leu Asp Lys Val Met Glu
        115                 120                 125

Thr Gln Ala Gln Val Asp Glu Leu Lys Gly Ile Met Val Arg Asn Ile
    130                 135                 140

Asp Leu Val Ala Gln Arg Gly Glu Arg Leu Glu Leu Leu Ile Asp Lys
145                 150                 155                 160

Thr Glu Asn Leu Val Asp Ser Ser Val Thr Phe Lys Thr Thr Ser Arg
                165                 170                 175

Asn Leu Ala Arg Ala Met Cys Met Lys Asn Leu Lys Leu Thr Ile Ile
            180                 185                 190

Ile Ile Ile Val Ser Ile Val Phe Ile Tyr Ile Ile Val Ser Pro Leu
        195                 200                 205

Cys Gly Gly Phe Thr Trp Pro Ser Cys Val Lys Lys
    210                 215                 220

<210> 7
<211> 564
<212> DNA
<213> Homo sapiens

<400> 7
atggcgattc tttttgctgt tgttgccagg gggaccacta tccttgccaa acatgcttgg 60
tgtggaggaa acttcctgga ggtgacagag cagattctgg ctaagatacc ttctgaaaat 120
aacaaactaa cgtactcaca tggcaattat ttgtttcatt acatctgcca agacaggatt 180
gtatatcttt gtatcactga tgatgatttt gaacgttccc gagcctttaa ttttctgaat 240
gagataaaga agaggttcca gactacttac ggttcaagag cacagacagc acttccatat 300
gccatgaata gcgagttctc aagtgtctta gctgcacagc tgaagcatca ctctgagaat 360
aagggcctag acaaagtgat ggagactcaa gcccaagtgg atgaactgaa aggaatcatg 420
gtcagaaaca tagatctggt agctcagcga ggagaaagat tggaattatt gattgacaaa 480
acagaaaatc ttgtggattc ttctgtcacc ttcaaaacta ccagcagaaa tcttgctcga 540

gccatgtgta tgaagaacct caag                                              564

<210> 8
<211> 188
<212> PRT
<213> Homo sapiens

<400> 8
Met Ala Ile Leu Phe Ala Val Val Ala Arg Gly Thr Thr Ile Leu Ala
1               5                   10                  15

Lys His Ala Trp Cys Gly Gly Asn Phe Leu Glu Val Thr Glu Gln Ile
            20                  25                  30

Leu Ala Lys Ile Pro Ser Glu Asn Asn Lys Leu Thr Tyr Ser His Gly
        35                  40                  45

Asn Tyr Leu Phe His Tyr Ile Cys Gln Asp Arg Ile Val Tyr Leu Cys
    50                  55                  60

Ile Thr Asp Asp Asp Phe Glu Arg Ser Arg Ala Phe Asn Phe Leu Asn
65                  70                  75                  80

Glu Ile Lys Lys Arg Phe Gln Thr Thr Tyr Gly Ser Arg Ala Gln Thr
                85                  90                  95

Ala Leu Pro Tyr Ala Met Asn Ser Glu Phe Ser Ser Val Leu Ala Ala
            100                 105                 110

Gln Leu Lys His His Ser Glu Asn Lys Gly Leu Asp Lys Val Met Glu
        115                 120                 125

Thr Gln Ala Gln Val Asp Glu Leu Lys Gly Ile Met Val Arg Asn Ile
    130                 135                 140

Asp Leu Val Ala Gln Arg Gly Glu Arg Leu Glu Leu Leu Ile Asp Lys
145                 150                 155                 160

Thr Glu Asn Leu Val Asp Ser Ser Val Thr Phe Lys Thr Thr Ser Arg
                165                 170                 175

Asn Leu Ala Arg Ala Met Cys Met Lys Asn Leu Lys
            180                 185

<210> 9
<211> 27

<212> DNA
<213> Artificial Sequence

<220>
<223> Description of Artificial Sequence:PCR PRIMERS

<400> 9
atggcgattc tttttgctgt tgttgcc                                          27


<210> 10
<211> 27
<212> DNA
<213> Artificial Sequence

<220>
<223> Description of Artificial Sequence:PCR PRIMERS

<400> 10
ctatttcttc acacagcttg gccatgt                                          27


<210> 11
<211> 27
<212> DNA
<213> Artificial Sequence

<220>
<223> Description of Artificial Sequence:PCR PRIMERS

<400> 11
atggcgattc tttttgctgt tgttgcc                                          27


<210> 12
<211> 27
<212> DNA
<213> Artificial Sequence

<220>
<223> Description of Artificial Sequence:PCR PRIMERS

<400> 12
cttattctca gagtgatgct tcagctg                                          27


<210> 13
<211> 27

<212> DNA
<213> Artificial Sequence

<220>
<223> Description of Artificial Sequence:PCR PRIMERS

<400> 13
atggcgattc tttttgctgt tgttgcc                                          27


<210> 14
<211> 32
<212> DNA
<213> Artificial Sequence

<220>
<223> Description of Artificial Sequence:PCR PRIMERS

<400> 14
atcctacttg aggttcttca tacacatggc tc                                    32


<210> 15
<211> 27
<212> DNA
<213> Artificial Sequence

<220>
<223> Description of Artificial Sequence:PCR PRIMERS

<400> 15
atgaatagcg agttctcaag tgtctta                                          27


<210> 16
<211> 27
<212> DNA
<213> Artificial Sequence

<220>
<223> Description of Artificial Sequence:PCR PRIMERS

<400> 16
ctatttcttc acacagcttg gccatgt                                          27


<210> 17
<211> 27

<212> DNA
<213> Artificial Sequence

<220>
<223> Description of Artificial Sequence:PCR PRIMERS

<400> 17
atgtcggcta ccgctgccac cgtcccg                                          27


<210> 18
<211> 27
<212> DNA
<213> Artificial Sequence

<220>
<223> Description of Artificial Sequence:PCR PRIMERS

<400> 18
ttaagagctg aagtaaacta tgatgat                                          27


<210> 19
<211> 303
<212> DNA
<213> Homo sapiens

<400> 19
atggcgattc tttttgctgt tgttgccagg gggaccacta tccttgccaa acatgcttgg 60
tgtggaggaa acttcctgga ggtgacagag cagattctgg ctaagatacc ttctgaaaat 120
aacaaactaa cgtactcaca tggcaattat ttgtttcatt acatctgcca agacaggatt 180
gtatatcttt gtatcactga tgatgatttt gaacgttccc gagcctttaa ttttctgaat 240
gagataaaga agaggttcca gactacttac ggttcaagag cacagacagc acttccatat 300
gcc                                                                303


<210> 20
<211> 66
<212> PRT
<213> Homo sapiens

<400> 20
Met Ala Ala Val Val Ala Arg Gly Thr Thr Ala Lys His Ala Trp Cys
  1               5                  10                  15

Gly Gly Asn Val Thr Ala Lys Ser Asn Asn Lys Thr Tyr Ser His Gly
             20                  25                  30

Asn Tyr His Tyr Cys Asp Arg Val Tyr Cys Thr Asp Asp Asp Arg Ser
        35                  40                  45

Arg Ala Asn Asn Lys Lys Arg Thr Thr Tyr Gly Ser Arg Ala Thr Ala
    50                  55                  60

Tyr Ala
65

<210> 21
<211> 27
<212> DNA
<213> Artificial Sequence

<220>
<223> Description of Artificial Sequence:PCR primer

<400> 21
ggatcctttc ttcacacagc ttggcca                                          27

<210> 22
<211> 27
<212> DNA
<213> Artificial Sequence

<220>
<223> Description of Artificial Sequence:PCR primer

<400> 22
ctatttcttc acacagcttg gccatgt                                          27